(19) Europäisches Patentamt
European Patent Office
Office européen des brevets

(11) Veröffentlichungsnummer: 0 437 187 A2

(12) EUROPÄISCHE PATENTANMELDUNG

(21) Anmeldenummer: 90811037.2

(22) Anmeldetag: 27.12.90

(51) Int. Cl.[5]: C07C 69/732, C07C 67/03, C07C 67/347

(30) Priorität: 11.01.90 CH 86/90
11.01.90 CH 87/90

(43) Veröffentlichungstag der Anmeldung: 17.07.91 Patentblatt 91/29

(84) Benannte Vertragsstaaten: DE FR GB IT

(71) Anmelder: CIBA-GEIGY AG
Klybeckstrasse 141
CH-4002 Basel(CH)

(72) Erfinder: Evans, Samuel, Dr.
Route des Charbonnieres 17
CH-1723 Marly(CH)
Erfinder: Dubs, Paul, Dr.
Route du Confin 14
CH-1723 Marly(CH)
Erfinder: Rusek, Milos
Tiefengrabenstrasse 49
CH-4102 Binningen(CH)
Erfinder: Mazour, Zdenek
Grütschweg 2
CH-4415 Lausen(CH)
Erfinder: Major, Arpad, Dr.
Chaistelweg 12
CH-4336 Kaisten(CH)

# (54) Verfahren zur Herstellung von Hydroxyphenylpropionsäureestern.

(57) Verfahren zur Herstellung von Verbindungen der allgemeinen Formel

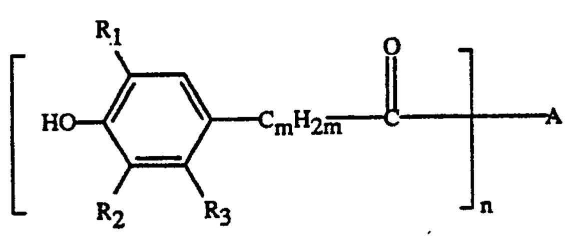

(I),

wobei
$R_1$ und $R_2$ gleich oder verschieden sind und Wasserstoff, $C_1$-$C_{18}$-Alkyl, Phenyl, $C_1$-$C_4$-alkylsubstituiertes Phenyl, $C_7$-$C_9$-Phenylalkyl, $C_5$-$C_{12}$-Cycloalkyl oder
$C_1$-$C_4$-alkylsubstituiertes $C_5$-$C_{12}$-Cycloalkyl sind,
$R_3$ Wasserstoff oder Methyl ist,
m O, 1, 2 oder 3 ist, und
n eine Zahl von 1 bis 4 oder 6 bedeutet, und
A die in der Beschreibung angegebenen Bedeutungen hat,
durch Umsetzung von Verbindungen der Formel II

EP 0 437 187 A2

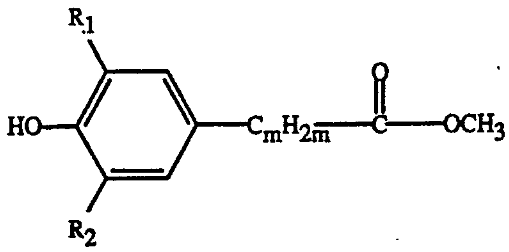

(II),

wobei m, $R_1$ und $R_2$ die obengenannte Bedeutung haben, mit Verbindungen der Formel III

$$A(-H)_n \qquad (III),$$

wobei A und n die in der Beschreibung genannten Bedeutungen haben, dadurch gekennzeichnet, dass die Reaktion in Gegenwart eines Katalysators, ausgeführt wird, der als aktives Material eine Alkalimetallverbindung der Formel IV

$M_mAn$ (IV)

worin
M Li, Na, K, Rb oder Cs,
m die Wertigkeit von An und
An ein Fluorid-, Hydroxid-, Phosphat-, Formiat-, Acetat- oder $-OR_5$-Rest bedeuten, und $R_5$ Alkyl mit 1 bis 4 C-Atomen oder einen Phenyl ist,
und als Träger ein alkalisches Material, das, gemessen in 10 Gew.-%-iger wässriger Suspension, einen pH-Wert von grösser als 10 aufweist, ausgewählt aus einer oder mehreren Substanzgruppen aus der Reihe der Erdalkalimetalloxide, -hydroxide, -aluminate oder -silikate, enthält.

## VERFAHREN ZUR HERSTELLUNG VON HYDROXYPHENYLPROPIONSÄUREESTERN

Die vorliegende Erfindung betrifft ein neues Verfahren zur Herstellung von 3-(4-Hydroxyphenyl)-propionsäureestern.

Es ist bekannt, Verbindungen der allgemeinen Formel

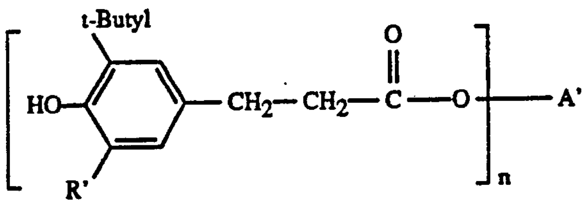

wobei R' Wasserstoff oder eine Alkylgruppe bedeutet, n eine Zahl von 2 bis 6 ist und A' eine acyclische oder cyclische aliphatische Gruppe bedeutet, durch Umsetzung eines entsprechenden Hydroxyphenylpropionsäureesters mit einer Verbindung der Formel

$$A'-(OH)_n,$$

wobei A' und n obengenannte Bedeutung haben, in Gegenwart eines Elementes der 2. Gruppe des periodischen Systems oder einer Verbindung eines dieser Elemente als Katalysator bei 170 bis 250°C herzustellen.

Das Verfahren, eine Umesterung, vermag jedoch nicht zu befriedigen, da zur Erzielung der angegebenen Umsätze und Ausbeuten hohe Reaktionstemperaturen und lange Reaktionszeiten Voraussetzung sind, wie z.B. der JP-A-62/53942 zu entnehmen ist. Weitere Umesterungsverfahren sind in US-A 3,644,482; 4,228,297; 3,644,482 und 3,285,855 sowie EP-A-102 920 beschrieben.

Es wurde nun gefunden, dass sich die Reaktionsführung durch Wahl eines geeigneten Katalysators noch verbessern lässt.

Gegenstand der vorliegenden Erfindung ist somit ein Verfahren zur Herstellung von Verbindungen der Formel I

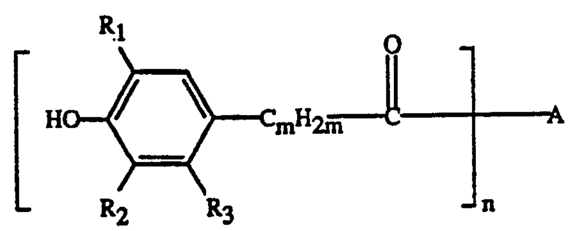 (I),

worin

$R_1$ und $R_2$ gleich oder verschieden sind und Wasserstoff, $C_1$-$C_{18}$-Alkyl, Phenyl, $C_1$-$C_4$-alkylsubstituiertes Phenyl, $C_7$-$C_9$-Phenylalkyl, $C_5$-$C_{12}$-Cycloalkyl oder $C_1$-$C_4$-alkylsubstituiertes $C_5$-$C_{12}$-Cycloalkyl sind,

$R_3$ Wasserstoff oder Methyl,

m 0, 1, 2 oder 3 und

n 1,2,3,4 oder 6 ist, wobei,

wenn n = 1 ist,

A $-OR_4$ ist, worin

$R_4$ $C_2$-$C_{45}$-Alkyl, $C_5$-$C_{12}$-Cycloalkyl, $C_2$-$C_{18}$-Alkenyl,

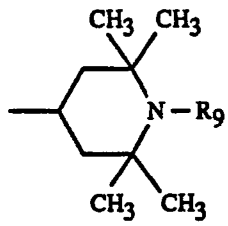

oder $-CH_2CH_2-XR_{5a}$ ist,
$R_9$ Wasserstoff, $C_1$-$C_8$-Alkyl, $C_3$-$C_5$-Alkenyl, Benzyl,

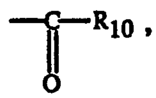

-O· oder $-OR_9$, ist,
worin
$R_9$, Wasserstoff, $C_1$-$C_{25}$-Alkyl oder

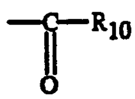

und
$R_{10}$ Wasserstoff oder $C_1$-$C_{20}$-Alkyl ist,
X -O-, -S- oder

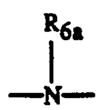

und

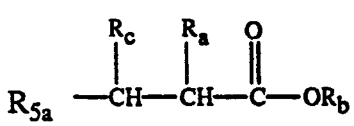

,

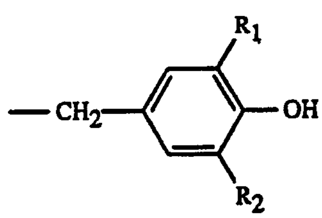

,

Wasserstoff, $C_1$-$C_{24}$-Alkyl, Phenyl, $C_5$-$C_{12}$-Cycloalkyl oder

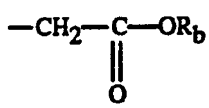

ist, worin
$R_a$ Wasserstoff oder Methyl,
$R_b$ Wasserstoff oder $C_1$-$C_{24}$-Alkyl und

$R_c$ Wasserstoff oder Methyl ist, mit der Massgabe, dass $R_a$ und $R_c$ nicht gleichzeitig Methyl sind, und

$R_{6a}$ $C_1$-$C_{18}$-Alkyl, Phenyl, mit einer oder mehreren Alkylgruppen mit insgesamt 1 bis 24 C-Atomen substituiertes Phenyl oder $C_5$-$C_8$-Cycloalkyl ist,

oder,

wenn n = 2 ist,

A $-O-C_xH_{2x}-O-$,

$-O-(CH_2CH_2O)_aCH_2CH_2O-$,

$-O-CH_2-CH_2-B-CH_2CH_2O-$,

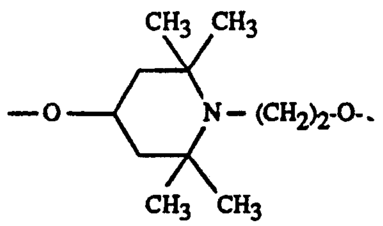 ,

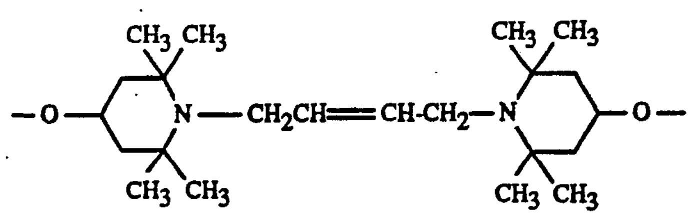

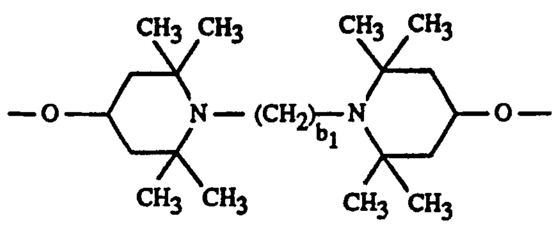 ,

$-O-CH_2CH=CHCH_2-O-$,

$-O-CH_2C\equiv CCH_2-O-$,

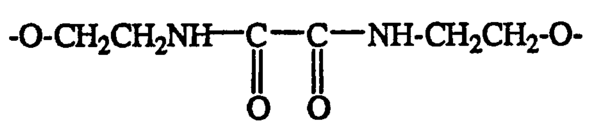 ,

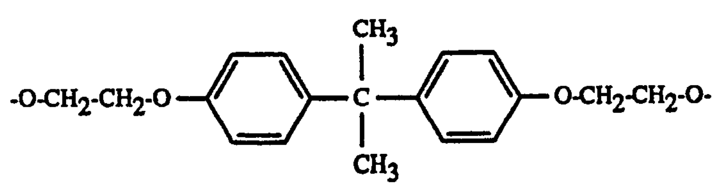 oder 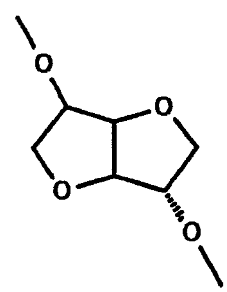

ist,

worin

a eine Zahl von 1 bis 30 und

x eine Zahl von 2 bis 20 ist,

B -S- , 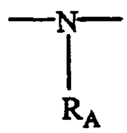 oder 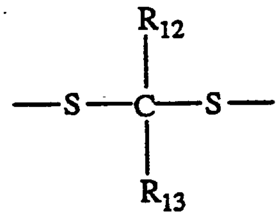

ist, worin
$R_A$ $C_1$-$C_{20}$-Alkyl, Phenyl oder mit einer oder mehreren Alkylgruppen mit insgesamt 1 bis 20 C-Atomen substituiertes Phenyl, Cyclohexyl oder

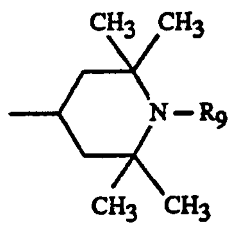

ist, worin $R_9$ die genannte Bedeutung hat, und
$R_{12}$ und $R_{13}$, unabhängig voneinander, Wasserstoff, $C_1$-$C_{12}$-Alkyl oder Phenyl sind oder
$R_{12}$ und $R_{13}$, mit dem C-Atom, an das sie gebunden sind, einen Cycloalkylring mit 5 bis 12 C-Atomen bilden, und
$b_1$ eine Zahl von 2 bis 10 ist,
oder,
wenn n = 3 ist,

A 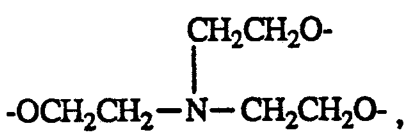 , 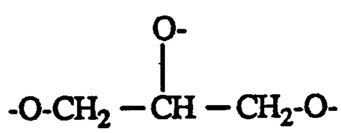 oder 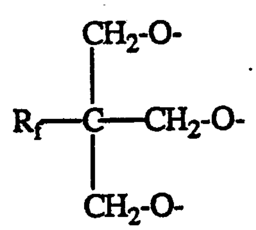

ist,

worin
$R_f$ $C_1$-$C_{24}$-Alkyl oder Phenyl ist,
oder,
wenn n = 4 ist,

A 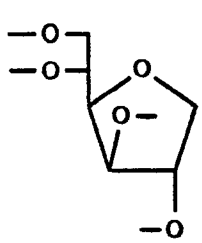 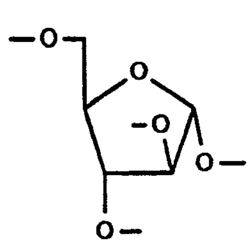 oder 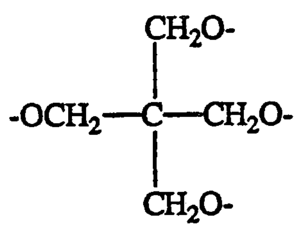

ist,
oder,
wenn n = 6 ist,

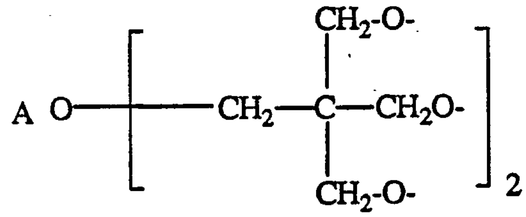

ist,
durch Umsetzung von Verbindungen der Formel II

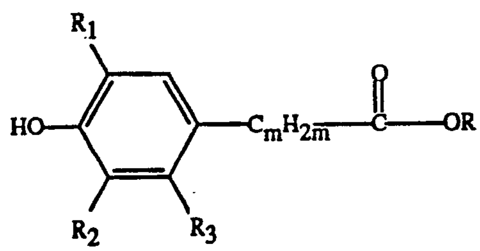 (II),

wobei m, $R_1$, $R_2$ und $R_3$ die obengenannte Bedeutung haben, und R Alkyl mit 1 bis 4 Kohlenstoffatomen ist, mit Verbindungen der Formel III

$$A-(-H)_n \qquad (III),$$

wobei A und n die obengenannte Bedeutung haben, dadurch gekennzeichnet, dass man die Reaktion in Gegenwart eines Katalysators ausführt, der als aktives Material eine Alkalimetallverbindung der Formel IV

$M_mAn$ (IV)

worin
M Li, Na, K,Rb oder Cs,
m die Wertigkeit von An und
An ein Fluorid-, Hydroxid-, Phosphat-, Formiat-, Acetat- oder $-OR_5$-Rest ist, und $R_5$ $C_1$-$C_4$-Alkyl oder Phenyl ist,
und als Träger ein alkalisches Material, das gemessen in 10 Gew.-%-iger wässriger Suspension, einen pH-Wert von grösser als 10 aufweist, ausgewählt aus einer oder mehreren Substanzgruppen der Reihe der Erdalkalimetalloxide, -hydroxide, -aluminate oder -silikate, enthält.

Gegenstand vorliegender Erfindung ist auch ein Verfahren zur Herstellung von Verbindungen, die als Zwischenprodukt im erfindungsgemässen Verfahren verwendet werden können, sowie ein Verfahren, das sowohl die Herstellung des Zwischenproduktes als auch dessen Umesterung zu den Verbindungen der Formel (I) umfasst.

Beispiele zu den Substituenten in den Formeln I, II und III sind nachfolgend angeführt.

Stellen $R_1$, $R_2$, und $R_{6a}$ beispielsweise Alkyl mit 1 bis 18 C-Atomen dar, so kann die Alkylgruppe geradkettig oder verzweigt sein und kann beispielsweise bedeuten: Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl oder 1-Methylundecyl. Bevorzugt sind Alkyl mit 1-12 C-Atomen, und besonders bevorzugt Alkyl mit 1-8 C-Atomen.

Für $R_1$ und $R_2$ sind die Alkylgruppen aus obiger beispielhafter Aufzählung mit 1 bis 8 C-Atomen als zweckmässig und mit 1 bis 4 C-Atomen als bevorzugt anzusehen. Ganz besonders bevorzugt für $R_1$ und $R_2$ wird die t-Butylgruppe. Weiters bevorzugt für $R_1$ ist $-CH_3$ und für $R_2$ t-Butyl oder für $R_1$ Isopropyl und für $R_2$ t-Butyl. Für $R_{6a}$ ist Alkyl mit 1- 12 C-Atomen bevorzugt. Für $R_3$ ist -H bevorzugt.

R bedeutet Alkyl mit 1 bis 4 Kohlenstoffatomen, wie Aethyl, Propyl, Butyl und insbesondere Methyl.

$R_4$ stellt beispielsweise geradkettiges oder verzweigtes Alkyl mit 1 bis 45 C-Atomen dar. Beispiele dafür sind jene, die für $R_1$ und $R_2$ angegeben sind, zuzüglich Eicosyl, Heneicosyl, Docosyl, Triacontyl usw. Bevorzugt für $R_4$ ist $C_1$-$C_{20}$-Alkyl und insbesondere $C_1$-$C_{18}$-Alkyl.

$R_A$ und $R_{10}$ können Alkyl mit 1 bis 20 C-Atomen bedeuten, wobei geradkettige oder verzweigte Alkylgruppen gemeint sind und auf die obengenannte Liste von Beispielen für $R_1$ und $R_2$, ergänzt durch das weitere Beispiel Eicosyl, hingewiesen wird. Bevorzugt werden Alkylgruppen mit 1 bis 12 C-Atomen.

$R_9$ bedeutet beispielsweise Alkyl mit 1 bis 8 C-Atomen, wobei die Alkylgruppen geradkettig oder verzweigt sein können und Methyl, Ethyl, Propyl, Isopropyl, n-Butyl, Isobutyl, tert.-Butyl, Pentyl, Isopentyl, Hexyl, Heptyl, Octyl, 2-Ethylhexyl, 2-Ethylbutyl, Isoamyl, 1 -Methylpentyl, 1,3-Dimethylbutyl, 1,1,3,3-Tetramethylbutyl, 1-Methylhexyl, Isoheptyl oder 1-Methylheptyl darstellen.

Ein zweckmässiger Rest für $R_9$ ist Alkyl mit 1 bis 4 C-Atomen, demnach beispielsweise Methyl, Ethyl, Propyl, Isopropyl, n-Butyl, Isobutyl oder tert.-Butyl.

$R_9$, kann Alkyl mit 1 bis 25 C-Atomen darstellen, Beispiele lassen sich sinngemäss der Liste für $R_1$ und $R_2$ entnehmen, ergänzt durch beispielsweise Eicosyl und Docosyl. Bevorzugt für $R_9$, ist $C_1$ bis $C_8$-Alkyl.

Stellt $R_b$, $R_f$ oder $R_{5a}$ eine Alkylgruppe mit 1 bis 24 C-Atomen dar, so zählen dazu die geradkettigen oder verzweigten Alkylgruppen wie sie beispielsweise für $R_1$ genannt wurden, ergänzt beispielhaft durch Eicosyl und Docosyl. Bevorzugt sind Alkylgruppen mit 1 bis 18 C-Atomen.

$R_{12}$ und $R_{13}$ stellen beispielsweise, unabhängig voneinander, Alkyl mit 1 bis 12 C-Atomen dar, wobei die Alkylgruppen geradkettig oder verzweigt sein können und in beispielhafter Aufzählung Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl oder 1-Methylundecyl sein können. Bevorzugt sind die entsprechenden Beispiele mit 1 bis 8 C-Atomen.

In vorzugsweiser Form kann $R_A$ eine $C_4$-$C_8$-Alkylgruppe sein und Beispiele für eine solche Gruppe sind n-Butyl, Isobutyl, tert.-Butyl, Pentyl, Isopentyl, Hexyl, Heptyl, Octyl, Isooctyl, 2-Ethylhexyl, 2-Ethylbutyl, 1-Methylpentyl, 1,3-Dimethylbutyl, 1,1,3,3-Tetramethylbutyl, 1-Methylhexyl, Isoheptyl oder 1-Methylheptyl.

Soweit i-$C_8H_{17}$ genannt wird, kann darunter auch eine Mischung von Isomeren verstanden werden.

Bedeuten $R_1$, $R_2$, $R_4$ oder $R_{5a}$ Cycloalkyl mit 5 bis 12 C-Atomen, so zählen beispielsweise dazu Cyclopentyl, Cyclohexyl, Cycloheptyl, Cyclooctyl, Cyclodecyl und Cyclododecyl. Bevorzugt wird Cyclohexyl.

$R_{6a}$ kann als Cycloalkyl mit 5 bis 8 C-Atomen die Bedeutung von Cyclopentyl, Cyclohexyl, Cycloheptyl oder Cyclooctyl haben.

Darüber hinaus stellen $R_1$ und $R_2$ auch $C_1$-$C_4$-alkylsubstituiertes Cycloalkyl mit 5 bis 12 C-Atomen dar. Beispiele dafür sind 2-oder 4-Methylcyclohexyl, Dimethylcyclohexyl, Trimethylcyclohexyl oder t-Butylcyclohexyl.

Bedeutet $R_{6a}$ ein mit einer oder mehreren, vorzugsweise mit einer oder zwei, Alkylgruppen mit insgesamt 1 bis 24 C-Atomen substituiertes Phenyl oder $R_A$ ein mit einer oder mehreren, vorzugsweise mit einer oder zwei, Alkylgruppen mit insgesamt 1 bis 20 C-Atomen substituiertes Phenyl, so gehören zu den Beispielen, die den Substituenten $R_{6a}$ und $R_A$ zugeordnet werden können, Methylphenyl, Dimethylphenyl, Trimethylphenyl, Ethylphenyl, Diethylphenyl, Isopropylphenyl, t-Butylphenyl, Di-t-butylphenyl, Methyl-di-t-butylphenyl, tert.-Octylphenyl und Di-tert.-octylphenyl.

Bedeutet $R_1$ und $R_2$ $C_1$-$C_4$-alkylsubstituiertes Phenyl, so sind Beispiele dafür Methylphenyl, Dimethylphenyl, Trimethylphenyl, Ethylphenyl, Diethylphenyl, Isopropylphenyl oder 6-Butylphenyl.

Schliesslich können $R_{12}$ und $R_{13}$, mit dem C-Atom an das sie gebunden sind, einen Cycloalkylring mit 5 bis 12 C-Atomen bilden. Dabei kann es sich in beispielhafter Nennung um einen Cyclopentyl-, Cyclohexyl-, Cycloheptyl-, Cyclooctyl-, Cyclodecyl- oder Cyclododecylring handeln. Bevorzugt ist ein Cyclohexylring.

Sind $R_1$ oder $R_2$ als Phenylalkyl mit 7 bis 9 C-Atomen bezeichnet, so sind Beispiele dafür Benzyl, Phenethyl, α-Methylbenzyl oder α,α-Dimethylbenzyl. Bevorzugt ist Benzyl. Ist $R_4$ eine Alkenylgruppe mit 2 bis 18 C-Atomen, so sind Beispiele dafür Vinyl, Propenyl, Allyl, Butenyl, Methallyl, Hexenyl, Decenyl oder Heptadecenyl.

Bedeutet m = 2, so wird damit beispielsweise die Gruppe $-CH_2-CH_2-$ beschrieben, bedeutet m = 3 so werden beispielsweise die Gruppen $-CH_2-CH_2-CH_2-$ oder

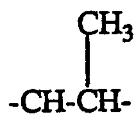

beschrieben.

In bevorzugten Verbindungen der Formel I, die nach dem Verfahren vorliegender Erfindung hergestellt werden können, bedeuten:

$R_1$ $C_1$-$C_{18}$-Alkyl, $C_5$-$C_{12}$-Cycloalkyl, Phenyl oder Benzyl,
$R_2$ Wasserstoff, $C_1$-$C_{18}$-Alkyl, $C_5$-$C_{12}$-Cycloalkyl, Phenyl oder Benzyl,
$R_3$ Wasserstfoff und
n eine Zahl von 1 bis 4 oder 6, und,
wenn n = 1 ist:
A $-OR_4$,
$R_4$ $C_2$-$C_{20}$-Alkyl, Cyclohexyl, $C_2$-$C_{18}$-Alkenyl oder $-CH_2-CH_2-XR_{5a}$,
X -O-, -S- oder

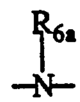,

$R_{5a}$ Wasserstoff, $C_1$-$C_{12}$-Alkyl, Phenyl, Cyclohexyl oder

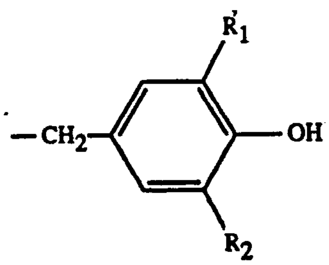,

$R_{6a}$ $C_1$-$C_{12}$-Alkyl, Phenyl oder mit einer oder mehreren Alkylgruppen mit insgesamt 1 bis 18 C-Atomen substituiertes Phenyl,
wenn n = 2 ist:
A $-O-C_xH_{2x}-O-$,
$-O-(CH_2CH_2O)_aCH_2CH_2O-$,
$-O-CH_2-CH_2-B-CH_2-CH_2O-$,

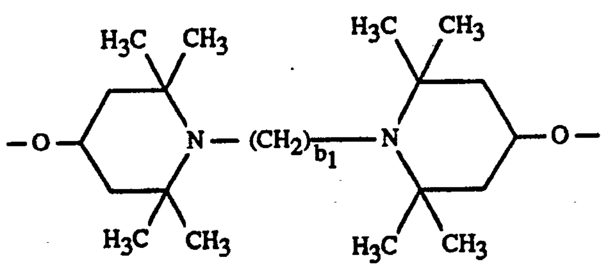

$-O-CH_2CH=CHCH_2-O-$,

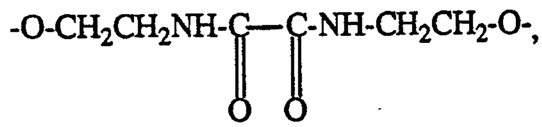

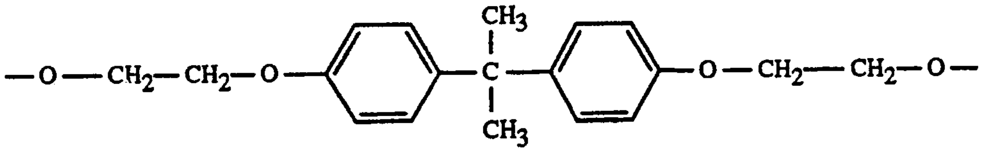

oder

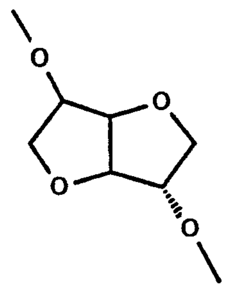
,

a eine Zahl von 1 bis 12,
x eine Zahl von 2 bis 12,
B -S- oder

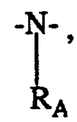
,

$R_A$ $C_1$-$C_{12}$-Alkyl, Phenyl, mit einer oder mehreren Alkylgruppen mit insgesamt 1 bis 18 C-Atomen substituiertes Phenyl oder Cyclohexyl,
$b_1$ eine Zahl von 2 bis 6,
wenn n = 3 ist:

A 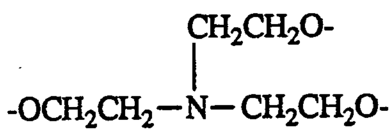 , 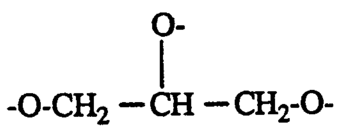 oder 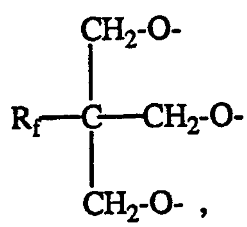 ,

$R_f$ $C_1$-$C_{12}$-Alkyl, und,
wenn n = 4 ist:

A 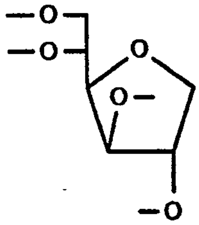 , 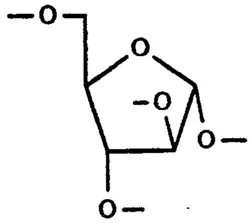 oder 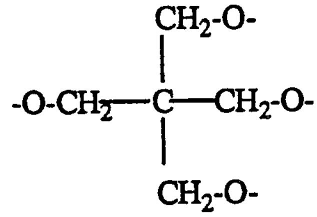 ,

und, wenn n = 6 ist:

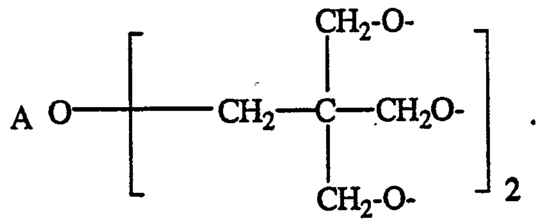

Das Verfahren nach der Erfindung ist besonders zweckmässig zur Herstellung von Verbindungen der Formel I, worin bedeuten:

$R_1$ $C_1$-$C_{18}$-Alkyl, Cyclohexyl oder Phenyl,
$R_2$ Wasserstoff, $C_1$-$C_8$-Alkyl, Cyclohexyl oder Phenyl,
$R_3$ Wasserstoff,
n 1,
A -$OR_4$,
$R_4$ $C_2$-$C_{18}$-Alkyl, Cyclohexyl, -$CH_2CH=CH_2$, -$(CH_2)_7$-$CH=CH$-$(CH_2)_7CH_3$,

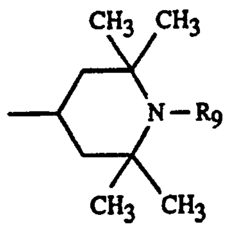

oder -$CH_2CH_2XR_{5a}$,
$R_9$ Wasserstoff, $C_1$-$C_4$-Alkyl, -O·, -O-Alkyl mit 1 bis 4 C-Atomen oder Cyclohexyl,
X -O-, -S-oder

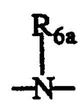 ,

$R_{5a}$ Wasserstoff, $C_1$-$C_{18}$-Alkyl oder Phenyl,
$R_{6a}$ $C_1$-$C_{12}$-Alkyl oder Phenyl, und
wenn n = 2:
A -O-$C_xH_{2x}$-O- oder -O-$(CH_2CH_2O)_aCH_2CH_2O$-,
x 2 bis 8, a 1,2,3 oder 4 und
wenn n = 3:

$$A - [OCH_2CH_2]_3N$$

und
wenn n = 4:

$$A\ C - [CH_2O]_4 - .$$

Das Verfahren nach der Erfindung wird zur Herstellung von Verbindungen der Formel I besonders bevorzugt, worin bedeuten:

$R_1$ tert.-Butyl,
$R_2$ Wasserstoff, Methyl oder tert.-Butyl,
$R_3$ Wasserstoff, und
wenn n = 1:
A-$OR_4$,

$R_4$ $C_2$-$C_{18}$-Alkyl, -$(CH_2)_7$-CH = CH-$(CH_2)_7CH_3$,

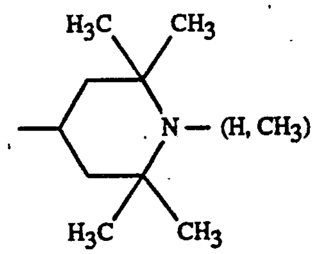

oder

-$CH_2CH_2$-$SR_{5a}$,

$R_{5a}$ Wasserstoff oder

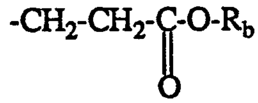 ,

und

wenn n = 2:

A -O-$C_xH_{2x}$-O-,

x 2 bis 8,

A -O-($CH_2$-$CH_2$-O-)$_a$-$CH_2$-$CH_2$-O-,

a 1 bis 4,

A -O-$CH_2$-$CH_2$-B-$CH_2$-$CH_2$-O- und

B -S-, 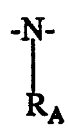 oder 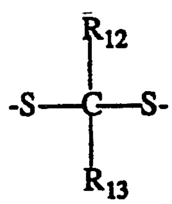 ,

$R_A$ $C_4$-$C_8$-Alkyl oder Phenyl,

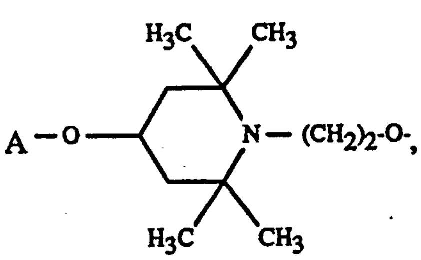

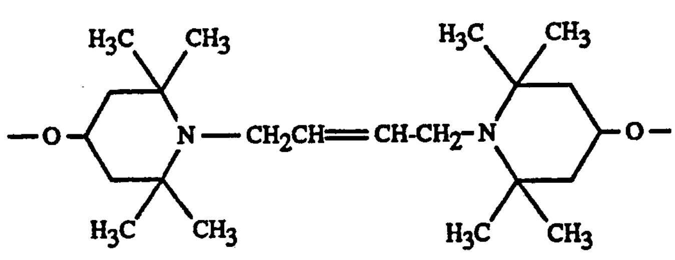

oder

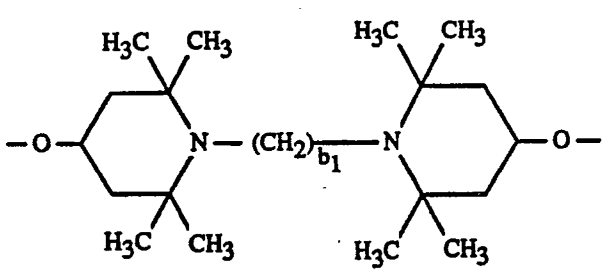 ,

$b_1$ 2 bis 6,
A $-O-CH_2-CH=CH-CH_2-O-$,
$-O-CH_2-C\equiv C-CH_2-O-$,

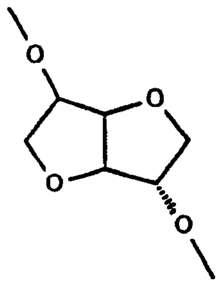
oder
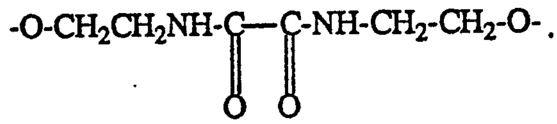

Das Verfahren nach der Erfindung ist ganz besonders bevorzugt zur Herstellung von Verbindungen der Formel I, worin bedeuten:
$R_1$ tert.-Butyl
$R_2$ Wasserstoff, Methyl oder tert.-Butyl,
$R_3$ Wasserstoff, und
wenn n = 1:
A $-OR_4$,
$R_4$ $C_1$-$C_{18}$-Alkyl;
oder zur Herstellung von Verbindungen der Formel I, worin bedeuten:
$R_1$ tert.-Butyl
$R_2$ Wasserstoff, Methyl oder tert.-Butyl,
$R_3$ Wasserstoff, und
wenn n = 2:
A $-O-C_xH_{2x}-O-$,
x 2 bis 6,
A $-O-(CH_2-CH_2-O)_a-CH_2-CH_2-O-$,
a 1,2 oder 3,
A $-O-CH_2-CH_2-B-CH_2-CH_2-O-$B -S- oder

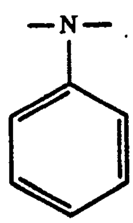
;

oder zur Herstellung von Verbindungen der Formel I, worin bedeuten:
$R_1$ tert.-Butyl,
$R_2$ Wasserstoff, Methyl oder tert.-Butyl,
$R_3$ Wasserstoff, und
wenn n = 1:
A $-OR_4$,
$R_4$ $C_2$-$C_{18}$-Alkyl, und
wenn n = 2:
A $-O-C_xH_{2x}-O-$ und x = 2 bis 6,
$-OCH_2CH_2-S-CH_2CH_2O-$ oder
$-O(CH_2CH_2O)_aCH_2CH_2-O-$
a 1 bis 4.

Bevorzugt sind insbesondere Verfahren zur Herstellung von Verbindungen der Formel I, wobei a 1 oder 2 ist.

Des weiteren bevorzugt sind Verfahren zur Herstellung von Verbindungen der Formel I, wobei bedeuten:

m 2,
$R_1$ tert.-Butyl,
$R_2$ Methyl oder tert.-Butyl,
$R_3$ Wasserstoff, und
wenn n = 3:

A 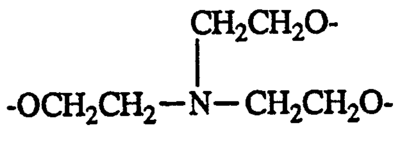 , 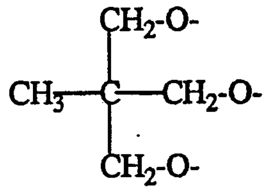 oder 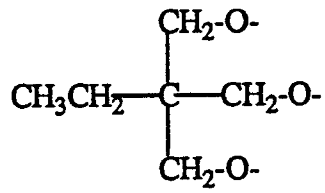 ,

und
wenn n = 4:

A 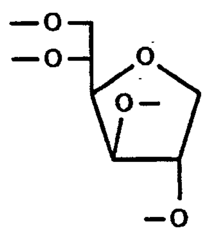 oder 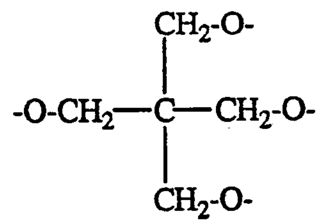 ,

und
wenn n = 6:

A 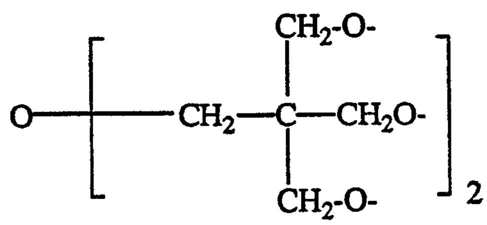

bedeutet.

Ganz besonders bevorzugt ist das erfindungsgemässe Verfahren zur Herstellung von Verbindungen des Typs:

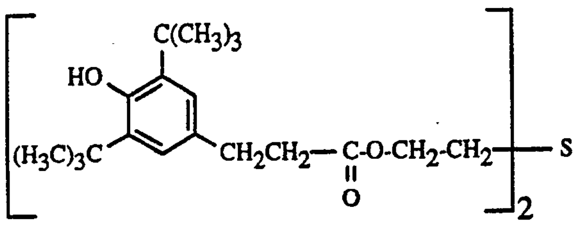
,

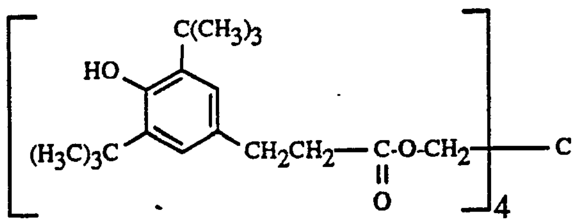
,

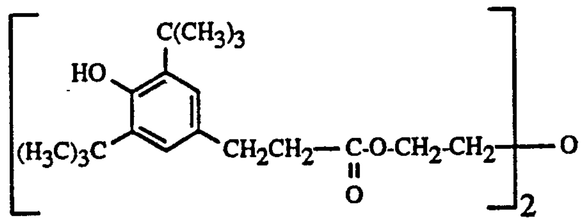
,

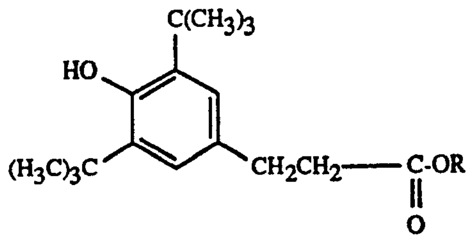
,

wobei

R $C_2$-$C_{18}$-Alkyl und insbesondere $C_{18}H_{37}$-Alkyl ist.

Die erfindungsgemässe Verbesserung des Verfahrens wird durch die Anwendung eines Katalysators erreicht, der als aktives Material eine Alkalimetallverbindung der Formel IV

$M_mAn^-$ (IV)

worin

M Li, Na, K, Rb oder Cs,

m die Wertigkeit von An und

An ein Fluorid-, Hydroxid-, Phosphat-, Formiat-, Acetat- oder $-OR_5$-Rest bedeutet, und $R_5$ $C_1$-$C_4$-Alkyl oder Phenyl ist,

und als Träger ein alkalisches Material, das, gemessen in 10 Gew.-%-iger wässriger Suspension, einen pH-Wert von grösser als 10 aufweist, ausgewählt aus einer oder mehreren Substanzgruppen aus der Reihe der Erdalkalimetalloxide, -hydroxide, -aluminate oder -silikate, enthält.

Die Trägersubstanzen aus der Reihe der genannten Erdalkalimetallverbindungen können in hydratisierter oder wasserfreier Form vorliegen, die hydratisierten Formen werden bevorzugt.

Zweckmässig sind Trägerkatalysatoren enthaltend die Oxide, Hydroxide, Aluminate oder Silikate der Erdalkalimetalle Mg, Ca, Sr und Ba oder deren Gemische als Träger.

Besonders zweckmässig als Träger sind die Verbindungen MgO, $Mg(OH)_2$, CaO, $Ca(OH)_2$, BaO, $Ba(OH)_2$, $Ba(OH)_2 \cdot 8H_2O$, geglühter Dolomit $MgO \cdot CaO$, geglühter und hydratisierter Dolomit $MgCa(OH)_4$, geglühter Barytokalzit $BaO \cdot CaO$, geglühter und hydratisierter Barytokalzit $BaCa(OH)_4$, Spinel $MgAl_2O_4$, $MgAl_2O_4 \cdot nH_2O$, $CaAl_2O_4$, $CaAl_2O_4 \cdot nH_2O$, Hydrokalumit $2Ca(OH)_2 \cdot Al(OH)_3nH_2O$, $Ca_2SiO_4$, Hillebrandit $Ca_2SiO_4 \cdot H_2O$, Foskagit und deren Gemische.

Bevorzugt sind CaO, MgO oder ein Gemisch aus diesen, z.B. hergestellt durch Brennen von Dolomit

$CaCO_3 \cdot MgCO_3$, als Träger.

Die Trägersubstanzen sind vorzugsweise im wesentlichen eisenfrei, d.h. der Gehalt an Eisen, auch in Form seiner Verbindungen, soll zweckmässig 10 ppm nicht überschreiten. Insgesamt sollen die Trägermaterialien von grosser Reinheit auch bezüglich weiterer Metalle, wie beispielsweise Kupfer, Blei und anderer Schwermetalle, sein. Der Kupfergehalt sollte zweckmässig unter 10 ppm liegen, ebenso wie der Gehalt an schweren Metallen, wie z.B. der Bleigehalt beispielsweise unter 10 ppm, und gesamthaft (alle Schwermetalle) beispielsweise unter 40 ppm liegen soll.

Desgleichen sind die Träger vorzugsweise weitgehend frei von Carbonatgruppen. Zweckmässig ist ein Carbonatgehalt von unter 0,1 Gew.-% einzuhalten. Sauerstoff übertragende Anionen, wie z.B. $MnO_4^-$, $CrO_4^-$, $AsO_4^{3-}$, $NO_3^-$ sollen zweckmässig höchstens zu je 100 ppm, zweckmässig gesamthaft zu höchstens 200 ppm vorhanden sein. Aktiver Sauerstoff darf zweckmässig 100 ppm nicht übersteigen. Starke acidische Anionen, z.B. $SO_4^{2-}$ oder $Cl^-$ sollten zweckmässig höchstens zu je 500 ppm und zweckmässig gesamthaft zu höchstens 1000 ppm vorliegen.

Zweckmässig sind Trägerkatalysatoren enthaltend als aktives Material die Hydroxide oder Fluoride der Alkalimetalle Na, K, Rb oder Cs.

Bevorzugtes aktives Material sind KOH, KF, NaOH, NaF oder CsF, besonders bevorzugt sind KOH oder KF.

Der Anteil des aktiven Materials ist beispielsweise 0,15 bis 30 Gew.-%, rechnerisch bezogen auf den wasserfreien Träger. Die Prozentangabe bezieht sich rechnerisch auf das entsprechende Alkalimetall allein, also ohne Berücksichtigung des jeweiligen Anions, während sich die Angabe für den Träger auf diesen insgesamt bezieht.

Zweckmässigerweise sind 0,15 bis 10 Gew.- % aktives Material vorgesehen und in bevorzugter Ausführungsform liegen 1 bis 10 Gew.- % aktives Material vor, jeweils bezogen auf das Alkalimetall und auf den wasserfreien Träger.

Für die Umsetzung von Verbindungen der Formel II mit Verbindungen der Formel III zu den entsprechenden Verbindungen der Formel I sind ganz besonders Katalysatoren geeignet, welche geglühtes und hydratisiertes CaO oder CaO und MgO, wobei letztere Mischung beispielsweise durch Glühen und anschliessendes Hydratisieren von Dolomit ($CaCO_3 \cdot MgCO_3$) erhältlich ist, und 5 bis 15, vorzugsweise 10 Gew.-%, K als KF oder KOH enthalten.

Besonders bevorzugte Katalysatoren sind KF als aktives Material auf $Ca(OH)_2$ als Träger, KOH auf $Ca(OH)_2$ und KF auf geglühtem und hydratisiertem Dolomit.

Die oben beschriebenen Katalysatoren sind besonders geeignet in einem Verfahren zur Herstellung von Verbindungen der allgemeinen Formel Ia

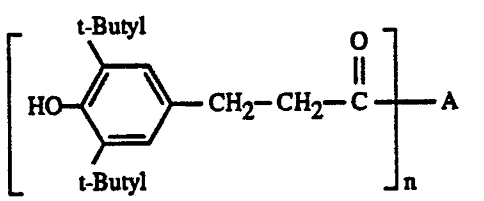

(Ia),

wobei A und n die obengenannten Bedeutungen haben, durch Umsetzung einer Verbindung der Formel IIa

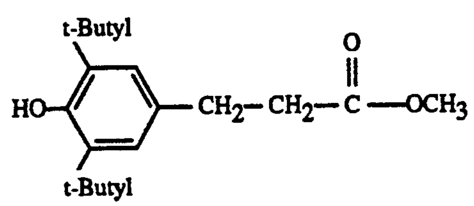

(IIa),

mit einer Verbindung

$$A\text{-}(H)_n$$

der Formel III, wie oben beschrieben.

Die vorliegende Erfindung betrifft auch die Verwendung der Katalysatoren, wie sie oben beschrieben werden, in einem Verfahren zur Umsetzung einer Verbindung der Formel II mit einer Verbindung der Formel III zu einer Verbindung der Formel I und bevorzugt zur Umsetzung einer Verbindung der Formel IIa mit einer Verbindung der Formel III zu einer Verbindung der Formel Ia. Die Bedeutung der Verbindungen der Formeln I, II, IIa und III lassen sich obiger Beschreibung entnehmen.

Katalysatoren nach der Erfindung mit Ca- oder Mg- oder Ba-haltigen Trägern sind beispielsweise dadurch erhältlich, dass durch thermische Behandlung in deren Oxide überführbare Verbindungen, wie $Ca(OH)_2$, $CaCO_3$, $Mg(OH)_2$, $MgCO_3$, Hydromagnesit $MgCO_3 \cdot Mg(OH)_2 \cdot nH_2O$, Dolomit $MgCO_3 \cdot CaCO_3$ oder $Ba(OH)_2 \cdot 8H_2O$ bei Temperaturen von 600 bis 1100 °C und vorzugsweise 900 bis 1100 °C, kalziniert werden.

Die entstehenden Träger sollen maximal 0,1 Gew.-% $CO_3^{2-}$ enthalten. Um eine Karbonisierung zu vermeiden, sollen die Träger zweckmässig beim Weiterbehandeln und Lagern durch ein Inertgas oder durch Vakuum vor der Umgebungsluft geschützt werden.

Ein derart vorbereiteter Träger kann, zweckmässig mit karbonatfreiem Wasser gelöscht werden, wobei sich das entsprechende Hydroxid bildet, das seinerseits mit einer wässrigen Lösung wenigstens eines Salzes von Li, Na, K, Rb oder Cs weiterbehandelt werden kann. Es ist auch möglich, den Träger mit einer wässrigen Lösung eines Salzes von Li, Na, K, Rb oder Cs direkt zu löschen, wobei sich nebeneinander, entsprechend dem Angebot an Wasser und Alkalimetallsalz die entsprechenden Hydroxide und weiteren Salze der Erdalkali-und Alkalimetalle ausbilden. Während des Löschverfahrens soll die Temperatur des Reaktionsgemisches zweckmässig 90 °C nicht übersteigen. Mit der zugegebenen Wassermenge kann man die Temperatur teilweise kontrollieren.

In der Regel soll der Katalysator nach dem Löschen in Form einer pastösen Masse vorliegen, in welcher die Wassermenge das Gewicht des Trägers zweckmässig um das 4-5fache nicht übersteigen darf. Vorzugsweise wird in inerter Atmosphäre, beispielsweise unter $N_2$, oder im Vakuum, getrocknet. Die Trocknungstemperaturen können zwischen 80 und 400, zweckmässig 350 °C, liegen, wobei es sich als zweckmässig erweist, unter Inertgas in erster Stufe während 3 bis 5 Stunden bei 90 °C und, in zweiter Stufe bis zu 350 °C ansteigend, 5 bis zu 24 Stunden oder während 3 bis 5 Stunden bei 150-200 °C, zweckmässig bei 160 °C, im Vakuum zu trocknen.

Danach weisen derart hergestellte Katalysatoren in der Regel eine keramikähnliche Struktur hoher Festigkeit auf und können auf das gewünschte Mass, beispielsweise durch Brechen und gegebenenfalls Mahlen, gebracht werden.

Wild der Katalysator als Suspension eingesetzt, ist es vorteilhaft, um eine spätere gute Abfiltrierbarkeit zu gewähren, auf eine Körnung von 0,2-0,5 mm zu zerkleinern. Wird eine Reaktion im Festbett angestrebt, ist eine Körnung von beispielsweise um 5 mm zweckdienlich.

Die Katalysatoren können auch durch physikalisches Mischen des Trägers mit einem aktiven Material erhalten werden.

Das Mischen kann auf an sich bekannte Weise erfolgen und entsprechend dem gewünschten Festigkeitsgrad oder der Körnung des fertigen Katalysators kann das Mischen beispielsweise auch in einer Kugelmühle erfolgen. Die Mischoperation wird zweckmässig unter Ausschluss von Luft, demnach entweder unter Inertgas, wie Stickstoff oder einem Edelgas, oder im Vakuum, durchgeführt. Das Mischen der Bestandteile erfolgt in diesem Fall in der Regel in trockenem Zustande, zweckmässig in hydratisierter Form, wie z.B. KOH mit $Ca(OH)_2$.

Der Katalysator kann in dispergierter Form in den Reaktanden vorliegen und wird nach erfolgter Umsetzung abfiltriert. Der durch Gebrauch erschöpfte Katalysator kann regeneriert und neu verwendet werden oder aber verworfen werden. Es ist auch möglich, den Katalysator in einem Festbett anzuordnen und die Reaktion beispielsweise in einem Strömungsreaktor durchzuführen (kontinuierliche Verfahrensweise).

Das Verfahren wird zweckmässig derart ausgeführt, dass die Ausgangsstoffe entweder in einem Lösungsmittel gelöst oder, soweit nicht flüssig, durch Temperaturerhöhung in Schmelze gebracht, umgesetzt werden.

Als Lösungsmittel kommen die an sich geläufigen Verbindungen und Gemische zur Anwendung, beispielsweise aromatische Kohlenwasserstoffe, wie Benzol, und alkylsubstituierte oder halogensubstituierte Benzole, insbesondere Toluol, Xylol oder Dichlorbenzol, hochsiedende aliphatische Kohlenwasserstoffe, wie hochsiedende Paraffine, aprotische Lösungsmittel, wie Dimethylformamid oder Dimethylanilin.

Der Katalysator wird dem Reaktionsgemisch beispielsweise in Mengen von 0,1 bis 10 Mol-%, zweckmässig von 1 bis 5 Mol-% und vorzugsweise von 2 bis 4 Mol-%, aktivem Material, bezogen auf die Verbindungen der Formel III, zugegeben.

Das Verhältnis von Verbindungen der Formel II oder IIa und die Verbindungen der Formel III im Reaktionsgemisch ist nicht kritisch und kann beispielsweise 0,8 bis 1,3 Mol der Verbindung II oder IIa pro Aequivalent der Verbindung III betragen. Zweckmässig sind 0,95 bis 1,2 Mol der Verbindung II oder IIa pro Aequivalent der Verbindung III und bevorzugt 1,0 bis 1,05 Mol der Verbindung II oder IIa pro Aequivalent der Verbindung III.

Die Reaktionstemperatur kann beispielsweise zwischen 110 und 250 °C, zweckmässig zwischen 130 und 195 °C und bevorzugt zwischen 130 und 170 °C liegen.

Die Umsetzung der Verbindungen der Formel II resp. IIa mit den Verbindungen der Formel III bis zum Erreichen optimaler Ausbeuten dauert in der Regel zwischen 1 und 5 Stunden, zweckmässig zwischen 1 und 4 Stunden und bevorzugt zwischen 1 und 3 Stunden.

Der Katalysator kann beispielsweise in pulveriger bis stückig gebrochener Form suspendiert im Reaktionsgemisch vorliegen. Eine Anwendung des Katalysators im Festbett ist eine weitere Möglichkeit der Verfahrensführung.

Nach Beendigung der Reaktion kann der Katalysator, sofern dieser in Suspension vorlag, abgetrennt, beispielsweise abfiltriert werden und das Endprodukt durch an sich bekannte Massnahmen, wie Kristallisation aus einem Lösungsmittel, wie Methanol, Isopropanol, Methanol-Wasser-Gemisch usw., isoliert werden.

Sofern notwendig kann das Reaktionsgemisch und/oder das Endprodukt mit einer Säure, z.B. Ameisensäure, Essigsäure, Schwefelsäure, Salzsäure usw., neutralisiert werden.

Die im erfindungsgemässen Verfahren eingesetzten Verbindungen der Formeln II und III sind bekannt oder können nach an sich bekannten Verfahren erhalten werden. In den eingangs zitierten Dokumenten sind solche Ausgangsverbindungen beschrieben.

Die erfindungsgemäss hergestellten Verbindungen der Formeln I und Ia stellen beispielsweise wertvolle Antioxidantien gegen oxidativen, thermischen oder actinischen Abbau von empfindlichen organischen Materialien dar. Solche Materialien sind beispielsweise synthetische Polymere oder funktionelle Flüssigkeiten, wie Schmierstoffe, Hydraulikflüssigkeiten oder Metallbearbeitungsflüssigkeiten etc.

Die vorliegende Erfindung betrifft auch ein neues Verfahren zur Herstellung von 3-(4-Hydroxyphenyl)-propionsäureestern der Formel V

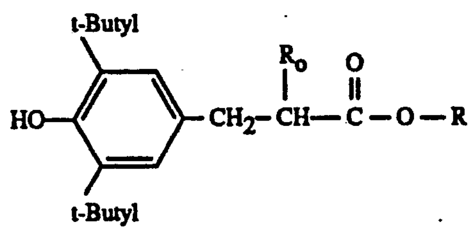

(V)

worin $R_0$ und R die unten angegebenen Bedeutungen besitzen.

Es ist bekannt, Verbindungen dieses Typs beispielsweise aus einem Hydroxyphenol und einem Acrylat in Gegenwart eines Katalysators herzustellen. Gemäss US-A-4,659,863 wird diese Reaktion in Gegenwart eines Alkalimetallkatalysators und eines Solubilisierungsmittels für den Katalysator ausgeführt.

Aus der US-A-3,840,585 ist ein derartiges Verfahren bekannt, das in Gegenwart einer katalytischen Menge von Hydriden, beispielsweise $NaBH_4$, $LiBH_4$ oder $Mg(AlH_4)_2$, ausgeführt werden.

Aus der US-A-3,364,250 ist beispielhaft ein Verfahren bekannt, gemäss dem aus Kaliummetall und tert.-Butylalkohol ein Katalysator hergestellt wird, in dessen Gegenwart dann genannte Reaktion abläuft.

Die US-A-3,247,240 beschreibt ein Verfahren vorstehend genannter Art, wobei die Umsetzung eines Dialkylhydroxyphenols mit einem Acrylsäureester in Gegenwart eines basischen Katalysators, beispielsweise aus der Reihe der Alkalimetallalkoxyde, der Alkalimetallhydroxyde und ferner der Erdalkalimetallalkoxide, erfolgt.

Aus der DE-A-23 64 126 ist ein Verfahren bekannt geworden zur Herstellung von Hydroxyalkylphenylderivaten durch Umsetzung von entsprechenden Phenolen mit Methylacrylat in Gegenwart eines Katalysators, wie beispielsweise einem Alkalimetallhydrid oder Alkalimetallhydroxid. Die Reaktionsmischung kann dann beispielsweise mit einem längerkettigen Alkohol mit einem zweiten Katalysator umgeestert werden.

Ein weiteres Verfahren ist aus der DE-A-23 64 121 bekannt, wonach beispielsweise 2,6-Di-tert.-butylphenol, n-Octadecylalkohol und Methylmethacrylat in Gegenwart von Natriummethylat direkt zum n-Octadecyl-$\beta$-(3,5-di-tert.-butyl-4-hydroxyphenyl)-propionat umgesetzt werden kann.

Es wurde nun gefunden, dass sich die Reaktionsführung durch Verwendung bestimmter Katalysatoren noch verbessern lässt. Nach dem Verfahren der Erfindung werden Verbindungen der allgemeinen Formel V

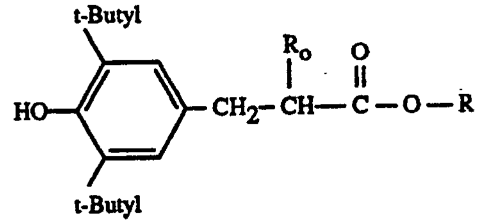

(V)

hergestellt, worin
$R_0$ Wasserstoff oder $C_1$-$C_4$-Alkyl, insbesondere Wasserstoff oder Methyl, und R $C_1$-$C_4$-Alkyl ist, durch Umsetzung von 2,6-Di-tert.-butylphenol mit einem Acrylsäureester der Formel VI

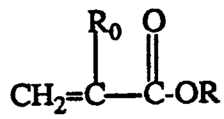

(VI),

worin $R_0$ und R die obengenannten Bedeutungen haben, dadurch gekennzeichnet, dass die Umsetzung in Gegenwart eines Katalysators ausgeführt wird, der als aktives Material eine Komponente der Formel IV

$M_mAn$ (IV)

worin
M Na, K, Rb oder Cs,
m die Wertigkeit von An und
An ein Fluorid-, Hydroxid- oder $-OR_5$-Rest ist, und $R_5$ $C_1$-$C_4$-Alkyl oder Phenyl ist, und als Träger ein alkalisches Material, das, gemessen in 10-Gew.-%-iger wässriger Suspension, einen pH-Wert von grösser als 10 aufweist, ausgewählt aus einer oder mehreren Substanzgruppen aus der Reihe der hydratisierten Erdalkalimetalloxide, -aluminate oder -silikate, enthält.

Bedeutet im aktiven Material An ein Rest $-OR_5$ und ist $R_5$ Alkyl mit 1 bis 4 C-Atomen, so sind Beispiele dafür Methyl, Ethyl, n-Propyl, i-Propyl, n-Butyl, sec.-Butyl oder tert.-Butyl.

Bevorzugt als Träger sind beispielsweise $Mg(OH)_2$, $Ca(OH)_2$, $Sr(OH)_2$, $Ba(OH)_2$, $MgAl_2O_4 \cdot nH_2O$, $CaAl_2O_4 \cdot nH_2O$, Hydrokalumit $2Ca(OH)_2 \cdot Al(OH)_3 \cdot nH_2O$, Hillebrandit $Ca_2SiO_4 \cdot H_2O$, geglühter und hydratisierter Dolomit $MgCa(OH)_4$, geglühter und hydratisierter Barytokalzit $CaBa(OH)_4$ und die Gemische genannter Substanzen untereinander. Die Trägersubstanzen sind auch in diesem Verfahren vorzugsweise im wesentlichen eisenfrei, d.h. der Gehalt an Eisen, auch in Form seiner Verbindungen, soll zweckmässig 10 ppm nicht überschreiten. Insgesamt sollen die Trägermaterialien von grosser Reinheit auch bezüglich weiterer Metalle, wie beispielsweise Kupfer, Blei und anderer Schwermetalle, sein. Der Kupfergehalt sollte zweckmässig unter 10 ppm liegen, ebenso wie der Gehalt an schweren Metallen, wie z.B. der Bleigehalt beispielsweise unter 10 ppm, und gesamthaft (alle Schwermetalle) beispielsweise unter 40 ppm liegen soll.

Desgleichen sind die Träger vorzugsweise weitgehend frei von Carbonatgruppen und insbesondere von Carbonationen. Zweckmässig ist ein Carbonatgehalt von unter 0,1 Gew.-% einzuhalten. Sauerstoff übertragende Anionen, wie z.B. $AsO_4^{3-}$, $NO_3^-$, $CrO_4^-$, $MnO_4^-$ sollen zweckmässig höchstens zu je 100 ppm, zweckmässig gesamthaft zu höchstens 200 ppm vorhanden sein. Aktiver Sauerstoff soll zweckmässig die Grenze von 100 ppm nicht überschreiten. Starke Anionen, wie acidische Anionen, z.B. $SO_4^{2-}$ oder $Cl^-$ sollten zweckmässig höchstens zu je 500 ppm und zweckmässig gesamthaft zu höchstens 1000 ppm vorliegen.

M bedeutet bevorzugt Na oder K.

Bevorzugtes aktives Material sind KOH, KF, NaOH, CsF oder NaF, besonders bevorzugt sind KOH oder KF.

Der Anteil des aktiven Materials ist beispielsweise 0,15 bis 30 Gew.-% bezogen auf den wasserfreien Träger. Die Prozentangabe bezieht sich rechnerisch auf das entsprechende Alkalimetallion allein, also ohne Berücksichtigung des jeweiligen Anions, während sich die Angabe für den wasserfreien Träger auf diesen insgesamt bezieht.

Zweckmässig sind 0,15 bis 10 Gew.-% aktives Material vorgesehen und in bevorzugter Ausführungsform liegen 1 bis 10 Gew.-% aktives Material vor, jeweils bezogen auf das Alkalimetallion und auf den

wasserfreien Träger.

Für die Umsetzung von Verbindungen der Formel VI mit 2,6-Di-tert.-butylphenol zu den entsprechenden Verbindungen der Formel I sind ganz besonders Katalysatoren geeignet, welche geglühtes und hydratisiertes CaO oder CaO und MgO, wobei letztere Mischung beispielsweise durch Glühen von Dolomit ($CaCO_3$, $MgCO_3$) und Hydratisieren erhältlich ist, und 5 bis 15, vorzugsweise 10 Gew.-%, KF oder KOH enthalten.

Besonders bevorzugte Katalysatoren sind KF als aktives Material auf $Ca(OH)_2$ als Träger, KOH auf $Ca(OH)_2$ und KF auf geglühtem und hydratisiertem Dolomit.

Die oben beschriebenen erfindungsgemäss einsetzbaren Katalysatoren sind besonders geeignet in einem Verfahren zur Herstellung von Verbindungen der allgemeinen Formel V, worin $R_0$ die Bedeutung von $-H$ oder $-CH_3$ hat und R die angegebene Bedeutung hat, und ganz besonders zur Herstellung von

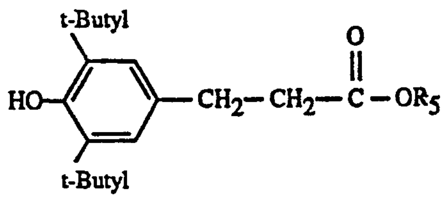 (Va), resp. 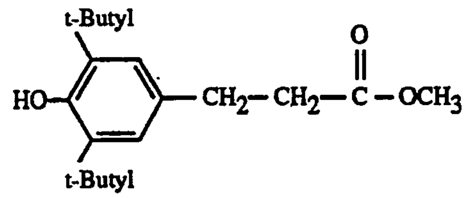 (Vb),

durch Umsetzung einer Verbindung der Formel VIa

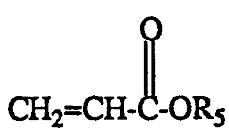 (VIa), resp. 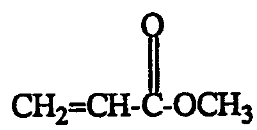 (VIb),

mit 2,6-Di-tert.-butylphenol, wie oben beschrieben.

Zum Umfange vorliegender Erfindung gehört auch die Verwendung der Katalysatoren, wie sie oben beschrieben werden, in einem Verfahren zur Umsetzung einer Verbindung der Formel VI mit 2,6-Di-tert.-butylphenol zu einer Verbindung der Formel V, zweckmässig zur Umsetzung einer Verbindung der Formel VIa mit 2,6-Di-tert.-butylphenol zu einer Verbindung der Formel Ia und bevorzugt zur Umsetzung einer Verbindung der Formel VIb mit 2,6-Di-tert.-butylphenol zu einer Verbindung der Formel Vb. Die Bedeutung der Verbindungen der Formeln V, Va, Vb, VI, VIa und VIb lassen sich obiger Beschreibung entnehmen.

Katalysatoren nach der Erfindung mit Ca-, Mg- oder Ba-haltigen Trägern sind beispielsweise dadurch erhältlich, dass CaO, MgO oder BaO oder durch thermische Behandlung in deren Oxid überführbare Verbindungen, wie $Ca(OH)_2$, $CaCO_3$, $Mg(OH)_2$, $MgCO_3$ oder $Mg(OH)_2$, bei Temperaturen von 600 bis 1100°C und vorzugsweise 900 bis 1100°C, kalziniert werden, oder durch kalzinieren von Dolomit oder Barytokalzit bei höherer Temperatur erhalten werden.

Die entstehenden Träger sollen zweckmässig weniger als 0,1 Gew.-% $CO_3^{2-}$ enthalten. Um eine Karbonisierung zu vermeiden, sollen die Träger zweckmässig beim Weiterbehandeln und Lagern durch ein Inertgas oder durch Vakuum vor der Umgebungsluft geschützt werden.

Ein derart vorbereiteter Träger kann, zweckmässig mit karbonatfreiem, Wasser gelöscht werden, wobei sich das entsprechende Hydroxid bildet, das seinerseits mit einer wässrigen Lösung wenigstens eines Salzes von Na, K, Rb oder Cs weiterbehandelt werden kann. Es ist auch möglich, den Träger mit einer wässrigen Lösung eines Salzes von Na, K, Rb oder Cs direkt zu löschen, wobei sich nebeneinander, entsprechend dem Angebot an Wasser und Alkalimetallsalz, die entsprechenden Hydroxide und weiteren Salze der Erdalkali- und Alkalimetalle ausbilden. Während des Löschverfahrens soll die Temperatur des Reaktionsgemisches zweckmässig 90°C nicht übersteigen. Mit der zugegebenen Wassermenge kann man die Temperatur teilweise kontrollieren.

Vorteilhaft liegt der Katalysator nach dem Löschen in Form einer pastösen Masse vor. Zu diesem Zweck kann die Wassermenge bis zum 4-5fachen des wasserfreien Trägers ausmachen. Die pastöse Masse wird, vorzugsweise in inerter Atmosphäre, beispielsweise unter $N_2$, oder im Vakuum, getrocknet. Die Trocknungstemperaturen können zwischen 80 und 400, zweckmässig bei 350°C, liegen, wobei es sich als zweckmässig erweist, unter Inertgas in erster Stufe während 3 bis 5 Stunden bei 90°C und, in zweiter Stufe bis zu 350°C ansteigend, 5 bis zu 24 Stunden oder während 3 bis 5 Stunden bei 150-200°C, zweckmässig bei 160°C, im Vakuum zu trocknen.

Danach weisen derart hergestellte Katalysatoren in der Regel eine keramikähnliche Struktur hoher Festigkeit auf und können auf das gewünschte Mass, beispielsweise durch Brechen und gegebenenfalls Mahlen, gebracht werden.

Wird der Katalysator als Suspension eingesetzt, ist es vorteilhaft, um eine spätere gute Abfiltrierbarkeit zu gewähren, auf eine Körnung von 0,2-0,5 mm zu zerkleinern; wird eine Reaktion im Festbett angestrebt, ist eine Körnung von beispielsweise um 5 mm zweckdienlich.

Die erfindungsgemässen Katalysatoren können auch durch physikalisches Mischen des Trägers mit einem aktiven Material erhalten werden.

Das Mischen kann auf an sich bekannte Weise erfolgen und entsprechend dem gewünschten Festigkeitsgrad oder der Körnung des fertigen Katalysators kann das Mischen beispielsweise auch in einer Kugelmühle erfolgen. Die Mischoperation wird zweckmässig unter Ausschluss von Luft, demnach entweder unter Inertgas, wie Stickstoff oder einem Edelgas, oder im Vakuum, durchgeführt. Das Mischen der Bestandteile erfolgt in diesem Fall in der Regel in trockenem hydrolysiertem Zustande.

Der Katalysator kann in dispergierter Form in den Reaktanden vorliegen und wird nach erfolgter Umsetzung abfiltriert. Die Zugabe eines Lösungsmittels, beispielsweise Toluol, kann den Filtrationsprozess fördern. Der durch Gebrauch erschöpfte Katalysator kann regeneriert und erneut verwendet werden oder aber verworfen werden. Die Regenerierung kann durch Auswaschen mit einem Lösungsmittel, z.B. Toluol, trocknen und befreien von Lösungsmittel und neu imprägnieren mit Alkalimetallsalzen und anschliessendem trocknen erfolgen. Es ist auch möglich, den Katalysator in einem Festbett anzuordnen und die Reaktion beispielsweise in einem Strömungsreaktor durchzuführen (kontinuierliche Verfahrensweise).

Das Verfahren wird zweckmässig derart ausgeführt, dass die Ausgangsstoffe entweder in einem Lösungsmittel gelöst oder, soweit nicht flüssig, durch Temperaturerhöhung in Schmelze gebracht, umgesetzt werden.

Als Lösungsmittel kommen die an sich geläufigen Verbindungen und Gemische zur Anwendung, beispielsweise aromatische Kohlenwasserstoffe, wie Benzol, und alkylsubstituierte oder halogensubstituierte Benzole, insbesondere Toluol, Xylol oder Dichlorbenzol, hochsiedende aliphatische Kohlenwasserstoffe, wie hochsiedende Paraffine, aprotische Lösungsmittel, wie Dimethylformamid oder Dimethylanilin, Dimethylsulfoxid oder Alkohole, wie tert.-Butanol.

Der Katalysator wird dem Reaktionsgemisch beispielsweise in Mengen von 0,1 bis 10 Mol-%, zweckmässig von 0,5 bis 5 Mol-%, besonders zweckmässig 1 bis 5 Mol-% und vorzugsweise von 2 bis 4 Mol-%, aktivem Material, bezogen auf 2,6-Di-tert.-butylphenol, zugegeben.

Das Verhältnis von Verbindungen der Formel VI und 2,6-Di-tert.-butylphenol im Reaktionsgemisch ist nicht kritisch und kann beispielsweise 0,8 bis 1,5 Mol der Verbindung VI pro Mol 2,6-Di-tert.-butylphenol betragen. Zweckmässig sind 0,95 bis 1,2 Mol der Verbindung VI pro Mol 2,6-Di-tert.-butylphenol und bevorzugt 1,0 bis 1,05 Mol der Verbindung VI pro Mol 2,6-Di-tert.-butylphenol.

Die Reaktionstemperatur kann beispielsweise zwischen 85 und 190 °C und zweckmässig zwischen 100 und 145 °C liegen.

Die Umsetzung der Verbindungen der Formel VI mit 2,6-Di-tert.-butylphenol bis zum Erreichen optimaler Ausbeuten dauert in der Regel zwischen 1 und 5 Stunden, zweckmässig zwischen 1 und 4 Stunden und bevorzugt zwischen 1 und 3 Stunden.

Der Katalysator kann beispielsweise in pulveriger bis stückig gebrochener Form suspendiert im Reaktionsgemisch vorliegen. Eine Anwendung des Katalysators im Festbett ist eine weitere Möglichkeit der Verfahrensführung.

Nach Beendigung der Reaktion kann der Katalysator, sofern dieser in Suspension vorlag, abgetrennt, beispielsweise abfiltriert werden und das Endprodukt durch an sich bekannte Massnahmen, wie Kristallisation aus einem Lösungsmittel, wie Methanol, Isopropanol, Methanol-Wasser-Gemisch usw., isoliert werden.

Sofern notwendig kann das Reaktionsgemisch und/oder das Endprodukt mit einer Säure, z.B. Ameisensäure, Essigsäure, Schwefelsäure, Salzsäure usw., neutralisiert werden.

Die erfindungsgemäss hergestellten Verbindungen der Formel V stellen beispielsweise wertvolle Zwischenprodukte dar, die erfindungsgemäss auch in einem Verfahren zur Herstellung von Verbindungen der allgemeinen Formel VII

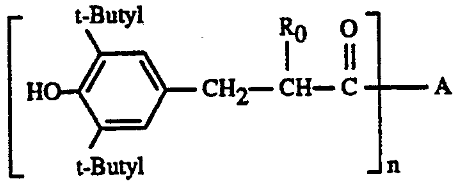

(VII),

verwendet werden können, worin
$R_0$ Wasserstoff oder $C_1$-$C_4$-Alkyl, insbesondere Wasserstoff oder Methyl, und
n 1, 2, 3, 4 oder 6 ist, worin,
wenn n = 1 ist,
A $-OR_4$ ist, worin
$R_4$ $C_2$-$C_{45}$-Alkyl, $C_5$-$C_{12}$-Cycloalkyl, $C_2$-$C_{18}$-Alkenyl,

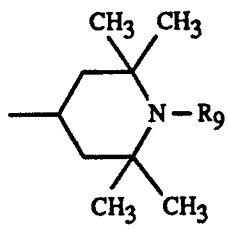

oder $-CH_2CH_2-XR_{5a}$ ist, worin
$R_9$ Wasserstoff, $C_1$-$C_8$-Alkyl,

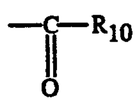,

$-O^{\cdot}$ oder $-OR_{9'}$ ist, worin
$R_{9'}$ Wasserstoff oder $C_1$-$C_{25}$-Alkyl oder

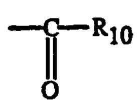,

und
$R_{10}$ Wasserstoff oder Alkyl mit 1 bis 20 C-Atomen ist,

X -O-, -S- oder

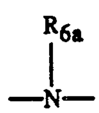,

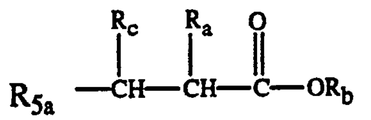 , 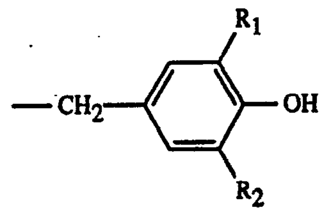 ,

Wasserstoff
$C_1$-$C_{24}$-Alkyl, Phenyl, $C_5$-$C_{12}$-Cycloalkyl oder

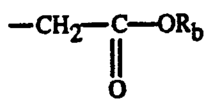

ist, worin
$R_a$ Wasserstoff oder Methyl,
$R_b$ Wasserstoff oder $C_1$-$C_{24}$-Alkyl und
$R_c$ Wasserstoff oder Methyl ist, mit der Massgabe, dass $R_a$ und $R_c$ nicht gleichzeitig Methyl sind,
und
$R_{6a}$ $C_1$-$C_{18}$-Alkyl, Phenyl, mit einer oder mehreren Alkylgruppen mit insgesamt 1 bis 24 C-Atomen substituiertes Phenyl oder $C_5$-$C_8$-Cycloalkyl ist,
oder,
wenn n = 2 ist,
A $-O-C_xH_{2x}-O-$,
$-O-(CH_2CH_2O)_aCH_2CH_2O-$,
$-O-CH_2-CH_2-B-CH_2CH_2O-$,

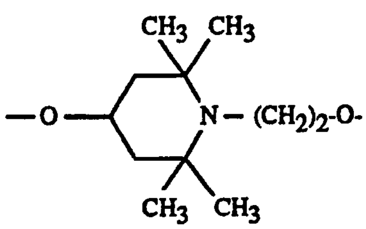 , 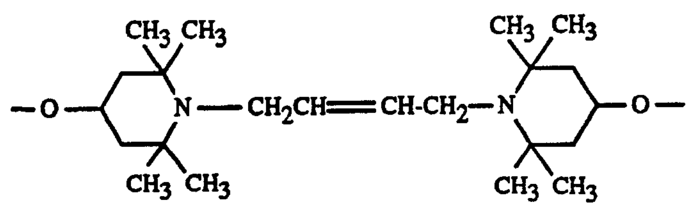

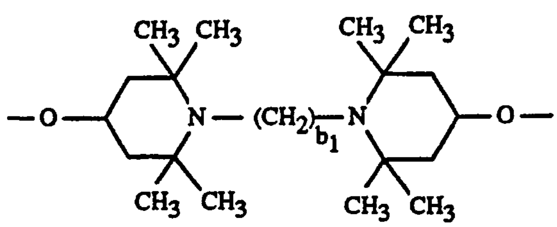

$-O-CH_2CH=CHCH_2-O-$, $-O-CH_2C\equiv CCH_2-O-$,

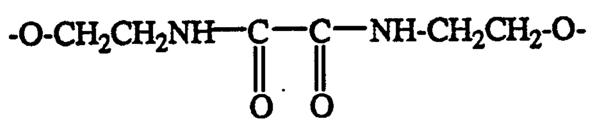 ,

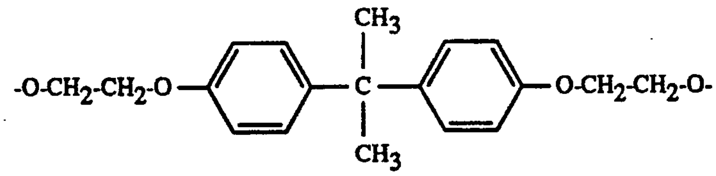 oder 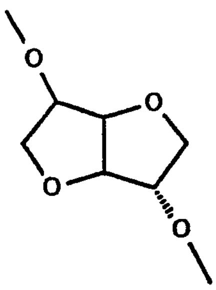

ist,
worin
a eine Zahl von 1 bis 30,
x eine Zahl von 2 bis 20 ist,

B -S- , 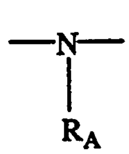 oder 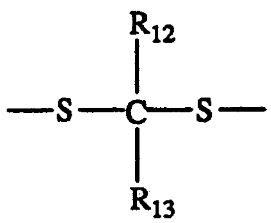

ist, worin
$R_A$ $C_1$-$C_{20}$-Alkyl mit 1 bis 20 C-Atomen, Phenyl oder mit einer oder mehreren Alkyl-gruppen mit insgesamt 1 bis 20 C-Atomen substituiertes Phenyl, Cyclohexyl oder

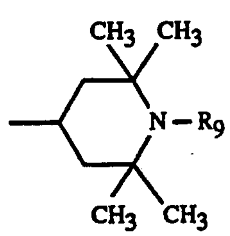

ist, wobei $R_9$ vorstehende Bedeutung hat, und
$R_{12}$ und $R_{13}$, unabhängig voneinander, Wasserstoff, $C_1$-$C_{12}$-Alkyl oder Phenyl sind oder
$R_{12}$ und $R_{13}$, mit dem C-Atom, an das sie gebunden sind, einen Cycloalkylring mit 5 bis 12 C-Atomen bilden, und
$b_1$ eine Zahl von 2 bis 10 ist,
oder,
wenn n = 3 ist,

A 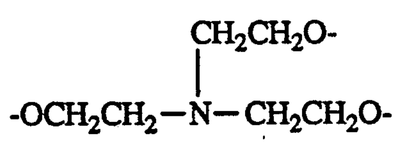, 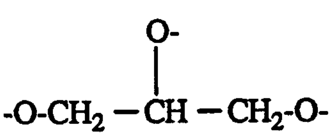 oder 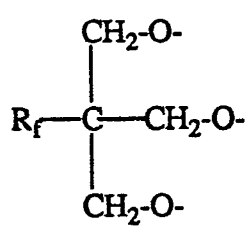

ist,
worin
$R_f$ $C_1$-$C_{24}$-Alkyl oder Phenyl darstellt,
oder,

wenn n = 4 ist,

A 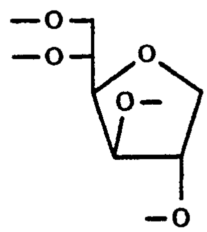 , 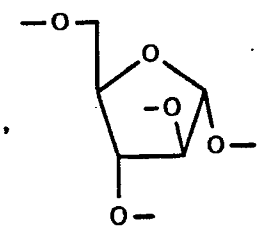 oder 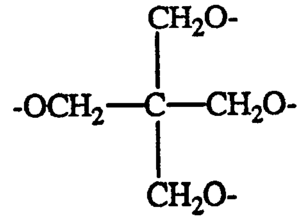

ist,
oder,
wenn n = 6 ist,

A 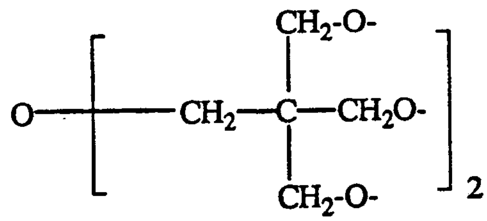

ist,
dadurch gekennzeichnet, dass man eine Verbindung der Formel VI

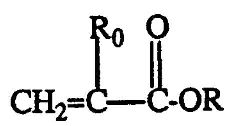 (VI),

worin $R_0$ Wasserstoff oder $C_1$-$C_4$-Alkyl und R $C_1$-$C_4$-Alkyl ist, mit 2,6-Di-tert.-butylphenol in erster Stufe zu einer Verbindung der Formel V umsetzt und letztere, ohne sie aus dem Reaktionsgemisch zu isolieren, in einer zweiten Stufe mit einer Verbindung der Formel III

$$A(-H)_n \qquad \text{(III)},$$

wobei A und n die genannte Bedeutung haben, umsetzt, wobei beide Stufen in Gegenwart eines Katalysators durchgeführt werden, der als aktives Material eine Komponente der Formel IV

$M_mAn$ (IV)

worin
M Na, K, Rb oder Cs,
m die Wertigkeit von An und
An ein Fluorid-, Hydroxid- oder -$OR_5$-Rest ist, und $R_5$ $C_1$-$C_4$-Alkyl oder ein Phenyl ist,
und als Träger ein alkalisches Material, das, gemessen in 10 Gew.-%-iger Suspension, einen pH-Wert von grösser als 10 aufweist, ausgewählt aus einer oder mehreren Substanzgruppen aus der Reihe der hydratisierten Erdalkalimetalloxide, -aluminate oder -silikate, enthält.

Die Bedeutungen des Substituenten A in Formel VII sind vorstehend aufgeführt, beispielhaft sind nachfolgend die Bedeutung der einzelnen Reste erwähnt.

Stellt $R_{6a}$ beispielsweise Alkyl mit 1 bis 18 C-Atomen dar, so kann die Alkylgruppe geradkettig oder verzweigt sein und kann beispielsweise bedeuten: Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl,

1,1,3,3-Tetramethylbutyl, 1-Methylhexyl, Isoheptyl, 1-Methylheptyl, 1,1,3-Trimethylhexyl oder 1-Methylundecyl. Bevorzugt sind Alkyl mit 1-12 C-Atomen, und besonders bevorzugt Alkyl mit 1-8 C-Atomen.

$R_4$ stellt beispielsweise geradkettiges oder verzweigtes Alkyl mit 2 bis 45 C-Atomen dar. Beispiele dafür sind jene, die für $R_{6a}$ angegeben sind, zuzüglich Eicosyl, Hemicosyl, Docosyl, Triacontyl usw. Bevorzugt für $R_4$ ist $C_5$-$C_{20}$-Alkyl und insbesondere $C_8$-$C_{18}$-Alkyl.

Stellt $R_4$ Alkyl mit 2, 3 resp. 4 C-Atomen dar, so wird bei der Umesterung der Verbindungen der Formel I von Verbindungen ausgegangen, deren $R_5$ Methyl, resp. Methyl oder Ethyl, resp. Methyl, Ethyl oder Propyl darstellen.

$R_A$ und $R_{10}$ können Alkyl mit 1 bis 20 C-Atomen bedeuten, wobei geradkettige oder verzweigte Alkylgruppen gemeint sind und auf die obengenannte Liste von Beispielen für $R_{6a}$, ergänzt durch das weitere Beispiel Eicosyl, hingewiesen wird. Bevorzugt werden Alkylgruppen mit 1 bis 12 C-Atomen.

$R_9$ bedeutet beispielsweise Alkyl mit 1 bis 8 C-Atomen, wobei die Alkylgruppen geradkettig oder verzweigt sein können und Methyl, Ethyl, Propyl, Isopropyl, n-Butyl, Isobutyl, tert.-Butyl, Pentyl, Isopentyl, Hexyl, Heptyl, Octyl, 2-Ethylhexyl, 2-Ethylbutyl, Isoamyl, 1 -Methylpentyl, 1,3-Dimethylbutyl, 1,1,3,3-Tetramethylbutyl, 1-Methylhexyl, Isoheptyl oder 1-Methylheptyl darstellen.

Ein zweckmässiger Rest für $R_9$ ist Alkyl mit 1 bis 4 C-Atomen, demnach beispielsweise Methyl, Ethyl, Propyl, Isopropyl, n-Butyl, Isobutyl oder tert.-Butyl.

$R_{9'}$ kann Alkyl mit 1 bis 25 C-Atomen darstellen, Beispiele lassen sich sinngemäss der Liste für $R_{6a}$ entnehmen, ergänzt durch beispielsweise Eicosyl und Docosyl. Bevorzugt für $R_{9'}$ ist $C_1$ bis $C_8$-Alkyl.

Stellt $R_b$, $R_f$ oder $R_{5a}$ eine Alkylgruppe mit 1 bis 24 C-Atomen dar, so zählen dazu die geradkettigen oder verzweigten Alkylgruppen wie sie beispielsweise für $R_{6a}$ genannt wurden, ergänzt beispielhaft durch Eicosyl und Docosyl. Bevorzugt sind Alkylgruppen mit 1 bis 18 C-Atomen.

$R_{12}$ und $R_{13}$ stellen beispielsweise, unabhängig voneinander, Alkyl mit 1 bis 12 C-Atomen dar, wobei die Alkylgruppen geradkettig oder verzweigt sein können und in beispielhafter Aufzählung Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl oder 1-Methylundecyl sein können. Bevorzugt sind die entsprechenden Beispiele mit 1 bis 8 C-Atomen.

In vorzugsweiser Form kann $R_A$ eine $C_4$-$C_8$-Alkylgruppe sein und Beispiele für eine solche Gruppe sind n-Butyl, Isobutyl, tert.-Butyl, Pentyl, Isopentyl, Hexyl, Heptyl, Octyl, Isooctyl, 2-Ethylhexyl, 2-Ethylbutyl, 1-Methylpentyl, 1,3-Dimethylbutyl, 1,1,3,3-Tetramethylbutyl, 1-Methylhexyl, Isoheptyl oder 1-Methylheptyl.

Soweit i-$C_8H_{17}$ genannt wird, kann darunter auch eine Mischung von Isomeren verstanden werden.

Bedeuten $R_6$ oder $R_{5a}$ Cycloalkyl mit 5 bis 12 C-Atomen, so zählen beispielsweise dazu Cyclopentyl, Cyclohexyl, Cycloheptyl, Cyclooctyl, Cyclodecyl und Cyclododecyl. Bevorzugt wird Cyclohexyl.

$R_{6a}$ kann als Cycloalkyl mit 5 bis 8 C-Atomen die Bedeutung von Cyclopentyl, Cyclohexyl, Cycloheptyl oder Cyclooctyl haben.

Bedeutet $R_{6a}$ ein mit einer oder mehreren, vorzugsweise mit einer oder zwei, Alkyl-gruppen mit insgesamt 1 bis 24 C-Atomen substituiertes Phenyl oder $R_A$ ein mit einer oder mehreren, vorzugsweise mit einer oder zwei, Alkylgruppen mit insgesamt 1 bis 20 C-Atomen substituiertes Phenyl, so gehören zu den Beispielen, die den Substituenten $R_{6a}$ und $R_A$ zugeordnet werden können, Methylphenyl, Dimethylphenyl, Trimethylphenyl, Ethylphenyl, Diethylphenyl, Isopropylphenyl, t-Butylphenyl, Di-t-butylphenyl, Methyl-di-t-butylphenyl, tert.-Octylphenyl und Di-tert-octylphenyl.

Schliesslich können $R_{12}$ und $R_{13}$, mit dem C-Atom an das sie gebunden sind, einen Cycloalkylring mit 5 bis 12 C-Atomen bilden. Dabei kann es sich in beispielhafter Nennung um einen Cyclopentyl-, Cyclohexyl-, Cycloheptyl-, Cyclooctyl-, Cyclodecyl- oder Cyclododecylring handeln. Bevorzugt ist ein Cyclohexylring.

Ist $R_4$ eine Alkenylgruppe mit 2 bis 18 C-Atomen, so sind Beispiele dafür Vinyl, Propenyl, Allyl, Butenyl, Methallyl, Hexenyl, Decenyl oder Heptadecenyl.

Bedeutet m = 2, so wird damit beispielsweise die Gruppe $-CH_2-CH_2-$ beschrieben, bedeutet m = 3 so werden beispielsweise die Gruppen $-CH_2-CH_2-CH_2-$ oder

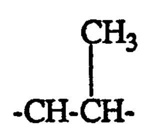

beschrieben.

In zweckmässigen Verbindungen der Formel VII, die nach dem Verfahren vorliegender Erfindung hergestellt werden können, bedeuten
n eine Zahl von 1 bis 4 oder 6, und
wenn n = 1:,
A $-OR_4$,
$R_4$ $C_2$-$C_{20}$-Alkyl, Cyclohexyl, $C_2$-$C_{18}$-Alkenyl oder $-CH_2-CH_2-XR_{5a}$,
X -O-, -S- oder

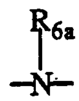,

$R_{5a}$ Wasserstoff, $C_1$-$C_{12}$-Alkyl, Phenyl, Cyclohexyl oder

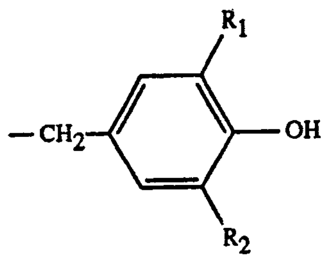,

und
$R_{6a}$ $C_1$-$C_{12}$-Alkyl, Phenyl oder mit einer oder mehreren Alkylgruppen mit insgesamt 1 bis 18 C-Atomen substituiertes Phenyl, und
wenn n = 2:
A $-O-C_xH_{2x}-O-$,
$-O-(CH_2CH_2O)_aCH_2CH_2O-$,
$-O-CH_2-CH_2-B-CH_2-CH_2O-$,

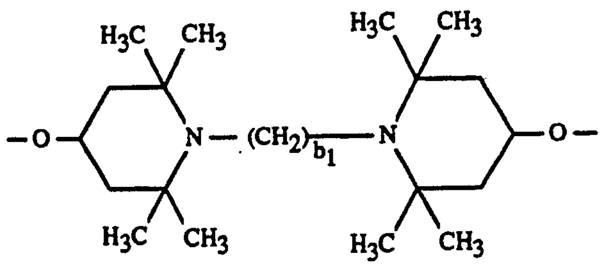

$-O-CH_2CH=CHCH_2-O-$,

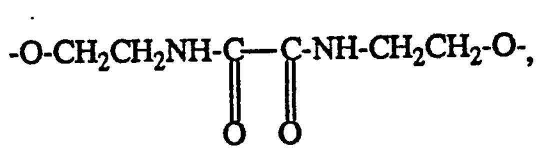,

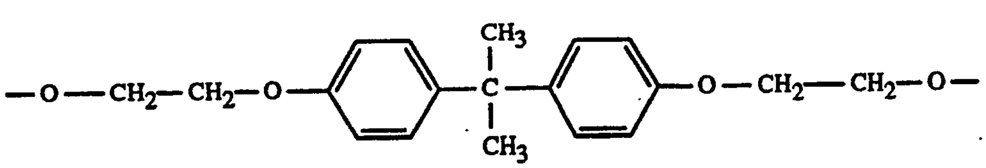

oder

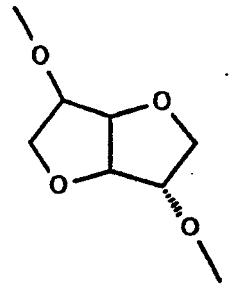,

a eine Zahl von 1 bis 12,
x eine Zahl von 2 bis 12,
B -S- oder

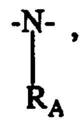,

$R_A$ $C_1$-$C_{12}$-Alkyl, Phenyl, mit einer oder mehreren Alkylgruppen mit insgesamt 1 bis 18 C-Atomen substituiertes Phenyl oder Cyclohexyl,
$b_1$ eine Zahl von 2 bis 6, und
wenn n = 3 ist,

A 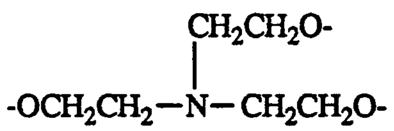, 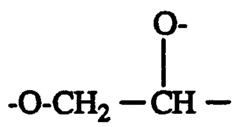 oder 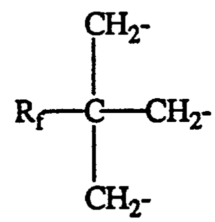,

$R_f$ $C_1$-$C_{12}$-Alkyl, und
wenn n = 4 ist,

A 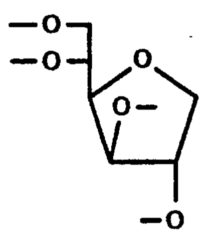, 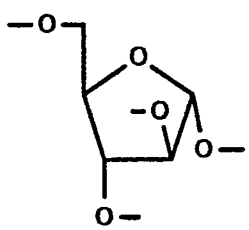 oder 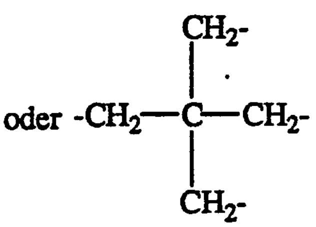,

und
wenn n = 6 ist,

A 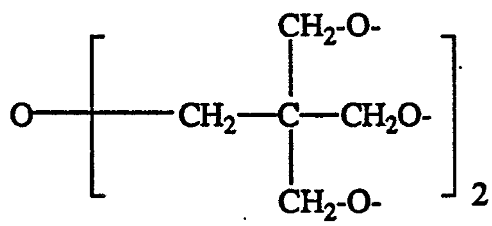.

Das Verfahren nach der Erfindung ist besonders zweckmässig zur Herstellung von Verbindungen der Formel VII, worin bedeuten:

wenn n 1 ist und
A $-OR_4$,
$R_4$ $C_2$-$C_{18}$-Alkyl, Cyclohexyl, $-CH_2CH=CH_2$, $-(CH_2)_7-CH=CH-(CH_2)_7CH_3$,

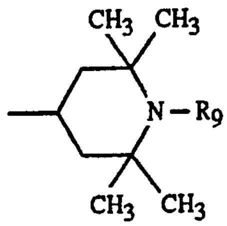 oder $-CH_2CH_2XR_{5a}$,

$R_9$ Wasserstoff, $C_1$-$C_4$-Alkyl, $-O^{\cdot}$, -O-Alkyl mit 1 bis 4 C-Atomen oder Cyclohexyl,
X -O-, -S- oder

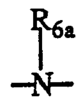,

$R_{5a}$ Wasserstoff, $C_1$-$C_{18}$-Alkyl oder Phenyl ist,
$R_{6a}$ $C_1$-$C_{12}$-Alkyl oder Phenyl, und
wenn n = 2:
A $-O-C_xH_{2x}-O-$ oder $-O-(CH_2CH_2O)_aCH_2CH_2O-$,
x 2 bis 8 und a 1,2,3 oder 4 und
wenn n = 3:

$$A -[OCH_2CH_2]_3N$$

und
wenn n = 4:

$$A\ C-[CH_2O]_4-.$$

Das Verfahren nach der Erfindung wird zur Herstellung von Verbindungen der Formel VII bevorzugt, worin bedeuten:
wenn n = 1 ist, wobei
A $-OR_4$,
$R_4$ $C_2$-$C_{18}$-Alkyl, $-(CH_2)_7-CH=CH-(CH_2)_7CH_3$,

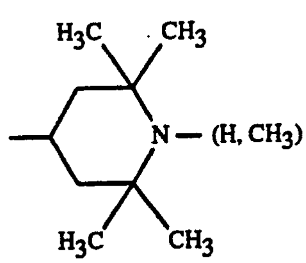

oder
$-CH_2CH_2-SR_{5a}$,
$R_{5a}$ Wassertoff oder

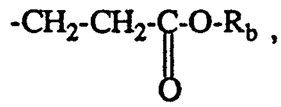

und

n = 2:

A $-O-C_xH_{2x}-O-$,

x 2 bis 8,

A $-O-(CH_2-CH_2-O-)_a-CH_2-CH_2-O-$,

a 1 bis 4,

A $-O-CH_2-CH_2-B-CH_2-CH_2-O-$

B -S-, 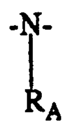

oder

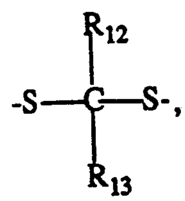

$R_A$ $C_4$-$C_8$-Alkyl oder Phenyl,

A 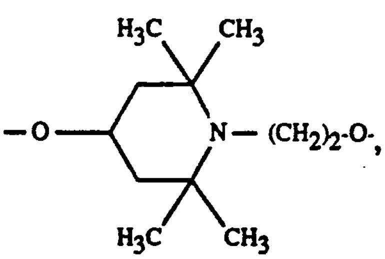

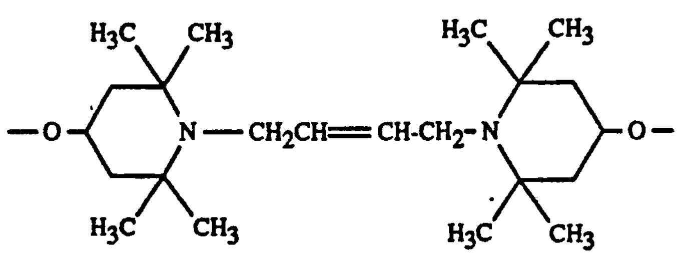

oder

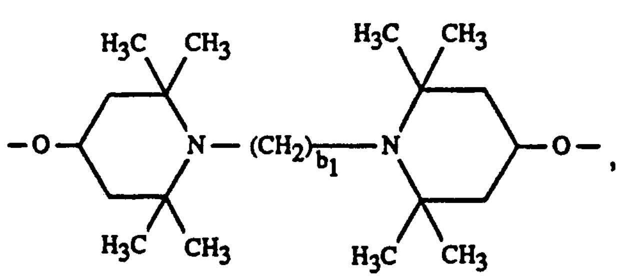

$b_1$ 2 bis 6,

A $-O-CH_2-CH=CH-CH_2-O-$,

$-O-CH_2-C \equiv C-CH_2-O-$,

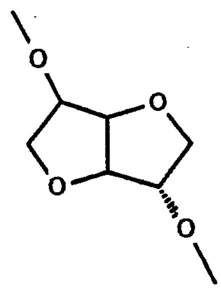
oder
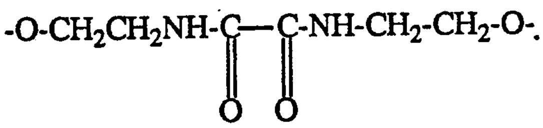

Das Verfahren nach der Erfindung ist besonders bevorzugt zur Herstellung von Verbindungen der Formel VII, worin bedeuten:
wenn n 1:
A $-OR_4$,
$R_4$ $C_2$-$C_{18}$-Alkyl,
oder zur Herstellung von Verbindungen der Formel III, worin bedeuten:
wenn n = 2:
A $-O-C_xH_{2x}-O-$,
x 2 bis 6,
A $-O-(CH_2-CH_2O)_aCH_2-CH_2-O-$
a 1, 2 oder 3,
A $-O-CH_2-CH_2-B-CH_2-CH_2-O-$
B -S- oder

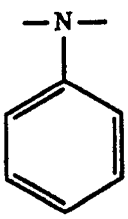

Bevorzugt sind insbesondere Verfahren zur Herstellung von Verbindungen der Formel VII, wobei n = 1 oder 2 ist.

Weiters bevorzugt sind Verfahren zur Herstellung von Verbindungen der Formel VII, wobei
$R_4$ Wasserstoff,
wenn n = 3:

A 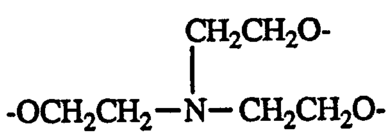 , 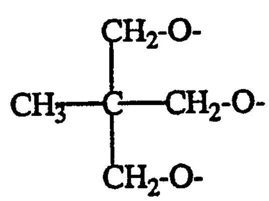 oder 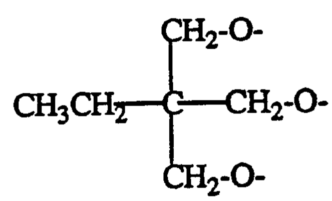 ,

und
wenn n = 4:

A 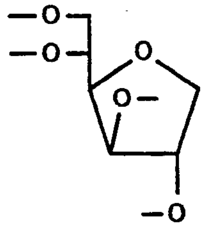 oder 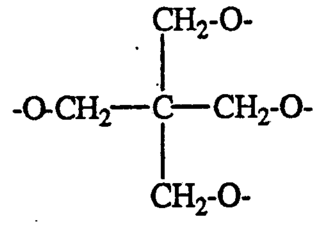 , und

n = 6:

A 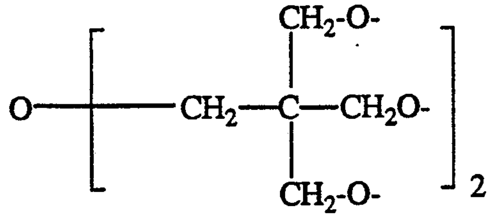 .

Ganz besonders bevorzugt ist das erfindungsgemässe Verfahren zur Herstellung von Verbindungen des Typs:

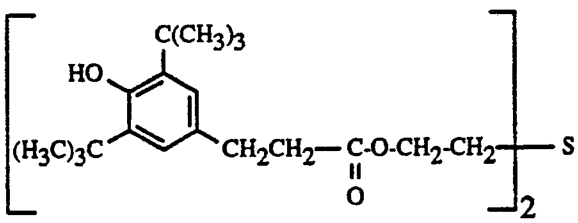
,

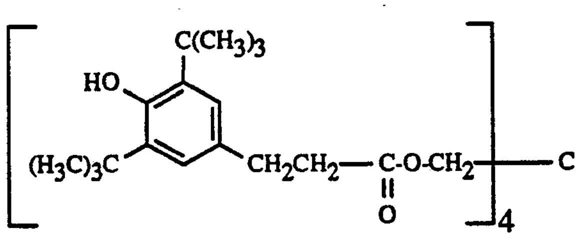
,

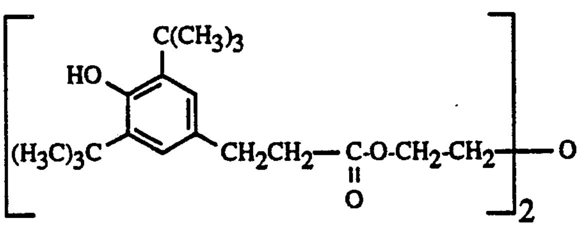
,

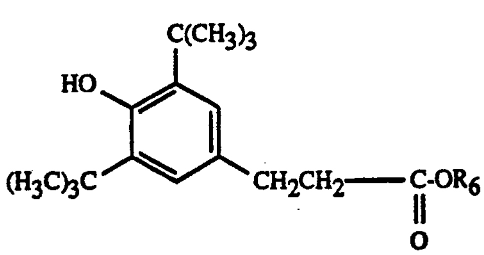
,

wobei

R die Bedeutung von $C_2$-$C_{18}$-Alkyl und insbesondere $C_{18}H_{37}$-Alkyl ist.

Das Verfahren wird zweckmässig derart ausgeführt, dass das Reaktionsgemisch aus der ersten Stufe in der Regel im gleichen Lösungsmittel gelöst bleibt, oder in Schmelze befindlich, mit der Verbindung der Formel III umgesetzt wird, dies ohne die Zwischenprodukte zu isolieren.

Das Verfahren ist an sich unkritisch gegenüber dem anzuwendenden Druck, deshalb wird vorzugsweise bei Normaldruck, im Vakuum oder im Hochvakuum gearbeitet.

Im Reaktionsgemisch der zweiten Stufe kann der Katalysator aus der ersten Stufe vorliegen und es kann auch zusätzlich weiterer Katalysator, wie vorstehend erwähnt zum Reaktionsgemisch zugefügt werden.

Der Katalysator soll im Reaktionsgemisch der zweiten Stufe beispielsweise in Mengen von 0,1 bis 10 Mol-%, zweckmässig von 1 bis 5 Mol-% und vorzugsweise von 2 bis 4 Mol-%, aktives Material, bezogen auf die Verbindungen der Formel V, vorliegen.

Das Verhältnis von Verbindungen der Formel V zu den Verbindungen der Formel III im Reaktionsgemisch ist nicht kritisch und kann beispielsweise 0,8 bis 1,3 Mol der Verbindung der Formel V pro Aequivalent der Verbindung III betragen. Zweckmässig sind 0,95 bis 1,2 Mol der Verbindung der Formel V pro Aequivalent der Verbindung III und bevorzugt 1,0 bis 1,05 Mol der Verbindung der Formel V pro Aequivalent der Verbindung III.

Die Reaktionstemperatur in der zweiten Stufe kann beispielsweise zwischen 110 und 240°C, zweckmässig zwischen 130 und 195°C und bevorzugt zwischen 135 und 190°C liegen.

Die Umsetzung in der zweiten Stufe der Verbindungen der Formel V mit den Verbindungen der Formel III bis zum Erreichen optimaler Ausbeuten dauert in der Regel zwischen 1 und 5 Stunden, zweckmässig zwischen 1 und 4 Stunden und bevorzugt zwischen 1 und 3 Stunden.

Auch in der zweiten Stufe kann der Katalysator, beispielsweise in pulveriger bis stückig gebrochener Form, suspendiert im Reaktionsgemisch vorliegen. Eine Anwendung des Katalysators im Festbett ist eine weitere Möglichkeit der Verfahrensführung.

Nach Beendigung der zweiten Reaktionsstufe kann der Katalysator, sofern dieser in Suspension vorlag, abgetrennt, beispielsweise abfiltriert werden und das Endprodukt durch an sich bekannte Massnahmen, wie Kristallisation aus einem Lösungsmittel, wie Methanol, Isopropanol, Methanol-Wasser-Gemisch, Isopropanol-Wasser-Gemisch, Benzol, Toluol, Xylol, Ligroin, n-Hexan usw., isoliert werden.

Sofern notwendig kann das Reaktionsgemisch und/oder das Endprodukt aus der zweiten Stufe mit einer Säure, z.B. Ameisensäure, Essigsäure, Schwefelsäure, Salzsäure usw., neutralisiert werden.

Die erfindungsgemäss hergestellten Verbindungen der Formel VII stellen beispielsweise wertvolle Antioxidantien gegen oxidativen, thermischen oder actinischen Abbau von empfindlichen organischen Materialien dar. Solche Materialien sind beispielsweise synthetische Polymere oder funktionelle Flüssigkeiten, wie Schmierstoffe, Hydraulikflüssigkeiten oder Metallbearbeitungsflüssigkeiten etc.

Die nachfolgenden Beispiele illustrieren die vorliegende Erfindung weiter. Alle Angaben in Prozenten oder Teilen beziehen sich auf das Gewicht, sofern nicht anders angegeben.

Beispiel 1: Herstellung eines Katalysators

Sehr reines Calciumoxid mit einem Gehalt an Ballaststoffen von Fe < 10 ppm, Cu < 10 ppm, schweren Metallen, (wie z.B. Pb) je< 10 ppm, gesamthaft < 40 ppm, Sauerstoff übertragende Anionen, wie z.B. $AsO_4^{3-}$, $NO_3^-$ und Peroxide je < 100 ppm, gesamthaft < 200 ppm, acidische Anionen, wie z.B. $SO_4^{2-}$, $Cl^-$ je < 500 ppm, gesamthaft < 1000 ppm, wird gemahlen.

Da dieser Rohstoff relativ hohe Carbonatgehalte aufweist, wird er vor der weiteren Verarbeitung bei 1100°C kalziniert, bis der $CO_3^{2-}$-Gehalt auf 0,1 Gew.-% gesunken ist (max. 5 Stunden).

Alle Operationen (das Kalzinieren eingeschlossen) werden unter Inertgas ($N_2$) oder im Vakuum durchgeführt.

In einen abschliessbaren Kneter aus rostfreiem Stahl, bestückt mit Temperiermantel und Austrageinrichtung, legt man 200-300 ml frisch destilliertes Wasser (ohne $CO_2$) vor. Der Kneter wird mit $N_2$ inertisiert und unter wirksamem Kneten trägt man portionenweise (die Temperatur soll 80°C nicht übersteigen) 100 g des geglühten Calciumoxides ins Wasser ein. Wenn die Reaktion beendet ist, giesst man zur Suspension eine Lösung von 16,7 g Kaliumfluorid in 20 ml destilliertem Wasser. Nach Durchkneten, bevor die Paste zäh zu werden beginnt, wird sie auf ein teflonbeschichtetes Stahlblech ausgetragen. Der derart zubereitete Katalysator unter Vakuum mit Zugabe eines Stickstoffstromes ($N_2$) getrocknet, wobei innerhalb 5 Stunden auf 160°C aufgeheizt wird. Der getrocknete Katalysator hat ein steinzeugähnliches Aussehen und liegt plattenförmig vor. Das Material wird dann gebrochen und auf eine Körnung von 0,2 - 0,5 mm gebracht. Bis zur Anwendung des Katalysators wird dieser unter Inertgas ($N_2$) gelagert. Der Katalysator hat einen Gehalt an K (berechnet auf wasserfreien Katalysator) von 10 Gew.-%, aktiver Sauerstoff ist weniger als 100 ppm

und $CO_3^{2-}$ weniger als 1 % enthalten.

Beispiel 2

Zu 376,4 g β-(3,5-Di-tert-butyl-4-hydroxyphenyl)-propionsäuremethylester werden 5 g Katalysator nach Beispiel 1 und 43,8 g Pentaerythritol gegeben und die Reaktionstemperatur wird stufenweise auf 190°C erhöht bei gleichzeitigem Anlegen eines Vakuums von unter 1000 Pa. Nach 4 Stunden ist die Umesterung beendet. Die Reaktionsmischung wird heiss filtriert, aus dem Filtrat die leichtflüchtigen Anteile abdestilliert und der Rückstand aus Methanol umkristallisiert. Der Umsatz beträgt rund 94 % bezogen auf die Ausgangsprodukte und es wird Pentaerythritol-tetrakis-[3-(3,5-Di-tert.-butyl-4-hydroxyphenyl)-propionat] der Formel

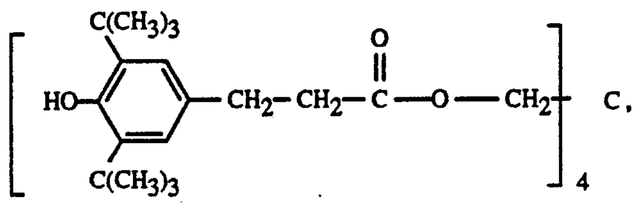

mit einem Schmelzpunkt von 118°C erhalten.

Beispiel 3: Herstellung von (3,5-Di-tert.-butyl-4-hydroxyphenyl)-propionsäuremethylester

Es werden 103,2 g (0,5 Mol) 2,6-Di-tert.-butylphenol und 47,3 g (0,55 Mol) Acrylsäuremethylester in Gegenwart von 5,8 g (2 Mol-% bezogen auf KF und Phenol) des Katalysators aus Beispiel 1 umgesetzt. Das 2,6-Di-tert.-butylphenol und der Katalysator werden im Reaktionsgefäss vorgelegt und dann das Reaktionsgefäss evakuiert. Das Reaktionsgefäss wird auf 65°C erwärmt, bis das 2,6-Di-tert.-butylphenol geschmolzen ist. Die Schmelze wird unter Vakuum gehalten und unter Rühren auf 115°C erhitzt. Bei dieser Temperatur wird der Acrylsäuremethylester innerhalb 2 Stunden zugetropft. Danach lässt man das Reaktionsgemisch weitere 2 Stunden unter Stickstoffatmosphäre ausreagieren. Der Katalysator wird abfiltriert und das Filtrat am Hochvakuum fraktionierend destilliert (124-128°C/$10^{-2}$ Torr).
Es werden 128,6 g entsprechend 88 % d.Th. praktisch weisser Kristalle mit einem Gehalt von 99,7 % (3,5-Di-tert.-butyl-4-hydroxyphenyl)-propionsäuremethylester erhalten.

Beispiel 4: Umsetzung von 2,6-Di-tert-butylphenol mit Acrylsäuremethylester zu Methyl-β-(3,5-di-tert-butyl-4-hydroxyphenyl)propionat und anschliessender Umesterung mit Pentaerythrit zu Pentaerythrityl-tetrakis-[β-(3,5-di-tert-butyl-4-hydroxyphenyl)-propionat].

Es werden 144,4 g (0,7 Mol) 2,6-Di-tert-butylphenol und 7,7 g (2 Mol-%) eines Trägerkatalysators aus 10 Gew.-% KOH auf geglühtem und hydratisiertem CaO als Träger in einen Kolben vorgelegt, mit Stickstoff beaufschlagt und auf 60°C aufgeheizt. Die leicht gelbliche Schmelze wird gerührt und unter Wasserstrahlvakuum wird die Temperatur auf 115°-120°C erhöht und während 1 Stunde gehalten. Nach aufheben des Vakuums werden 66,3 g (0,77 Mol) Acrylsäuremethylester innerhalb von 2 Stunden zum Reaktionsgemisch getropft. Bei exothermer Reaktion entwickelt sich ein Ueberdruck der durch einen Kühler entweichen kann. Bei Normaldruck wird eine weitere Stunde ausreagieren gelassen, wobei das Reaktionsgemisch mit $N_2$ überschichtet wird.
Eine Kontrolle mit dem Gaschromatographen ergibt eine Ausbeute von 98 %. Zum entstandenen Reaktionsgemisch werden 18,4 g (0,135 Mol) Pentaerythrit gegeben und das Reaktionsgefäss wird evakuiert und innerhalb 1 1/2 Stunden wird die Temperatur auf 190°C erhöht, wobei sich ca. 16 g Methanol abscheiden. Es wird weitere zwei Stunden bei 190°C ausreagieren gelassen und dann die 190°C heisse Schmelze über eine Nutsche abgesaugt. Die Schmelze wird am Hochvakuum bis auf 220°C erhitzt, wobei nicht reagiertes Methyl-β-(3,5-di-tert-butyl-4-hydroxyphenyl)-propionat abdestilliert wird. Der Rückstand wird über Methanol umkristallisiert und es werden 146,3 g (92 % der Theorie) Pentaerythrityl-tetrakis-[β-(3,5di-tert-butyl-4-hydroxyphenyl)-propionat] als weisses Pulver mit dem Smp. von 118°C erhalten.

**Patentansprüche**

1. Verfahren zur Herstellung von Verbindungen der allgemeinen Formel

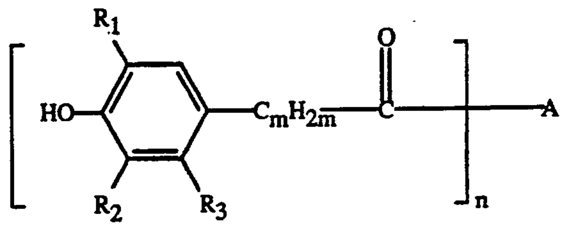

(I),

worin

$R_1$ und $R_2$ gleich oder verschieden sind und Wasserstoff, $C_1$-$C_{18}$-Alkyl, Phenyl, $C_1$-$C_4$-alkylsubstituiertes Phenyl, $C_7$-$C_9$-Phenylalkyl, $C_5$-$C_{12}$-Cycloalkyl oder $C_1$-$C_4$-alkylsubstituiertes $C_5$-$C_{12}$-Cycloalkyl sind,

$R_3$ Wasserstoff oder Methyl,

m O,1,2 oder 3 und

n 1,2,3,4 oder 6 ist, wobei,

wenn n = 1 ist,

A $-OR_4$ ist, worin

$R_4$ $C_2$-$C_{45}$-Alkyl, $C_5$-$C_{12}$-Cycloalkyl, $C_2$-$C_{18}$-Alkenyl,

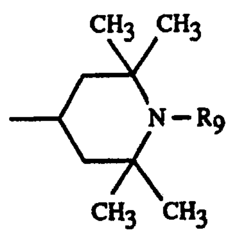

oder $-CH_2CH_2XR_{5a}$ ist,

$R_9$ Wasserstoff, $C_1$-$C_8$-Alkyl, $C_3$-$C_5$-Alkenyl, Benzyl,

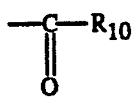

,

$-O^{\cdot}$ oder $-OR_{9'}$ ist,

worin

$R_{9'}$ Wasserstoff, $C_1$-$C_{25}$-Alkyl oder

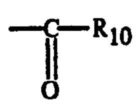

und

$R_{10}$ Wasserstoff oder $C_1$-$C_{20}$-Alkyl ist,

X -O-, -S- oder

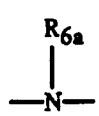

und

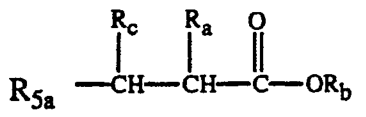 , 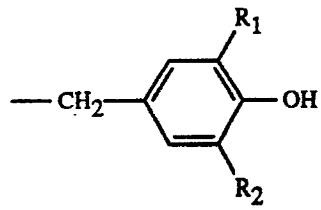 ,

Wasserstoff, $C_1$-$C_{24}$-Alkyl, Phenyl, $C_5$-$C_{12}$-Cycloalkyl oder

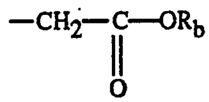

ist, worin
$R_a$ Wasserstoff oder Methyl
$R_b$ Wasserstoff oder $C_1$-$C_{24}$-Alkyl und
$R_c$ Wasserstoff oder Methyl ist, mit der Massgabe, dass $R_a$ und $R_c$ nicht gleichzeitig Methyl sind,
und
$R_{6a}$ $C_1$-$C_{18}$-Alkyl, Phenyl, mit einer oder mehreren Alkylgruppen mit insgesamt 1 bis 24 C-Atomen substituiertes Phenyl oder $C_5$-$C_8$-Cycloalkyl ist,
oder,
wenn n = 2 ist,
A $-O-C_xH_{2x}-O-$,
$-O-(CH_2CH_2O)_aCH_2CH_2O-$,
$-O-CH_2-CH_2-B-CH_2CH_2O-$,

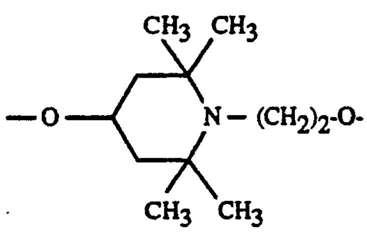 , 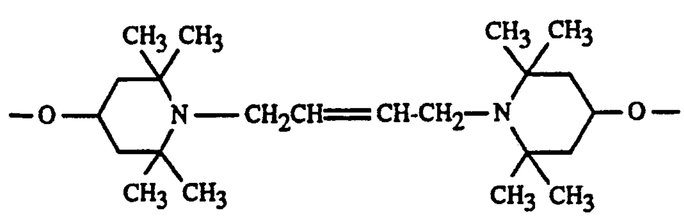

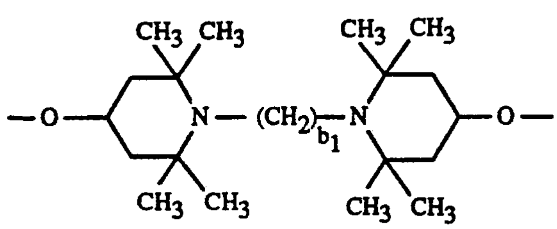 ,

$-O-CH_2CH=CHCH_2-O-$,
$-O-CH_2C\equiv CCH_2-O-$,

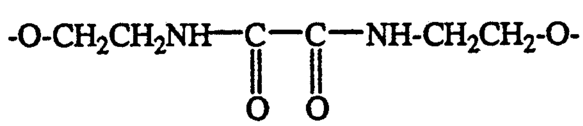 ,

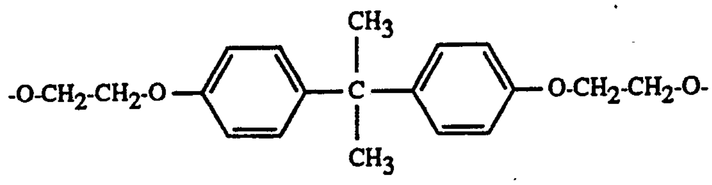 oder 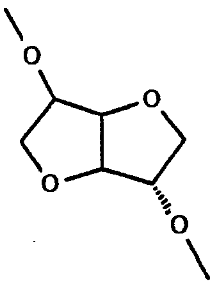 ist,

worin
a eine Zahl von 1 bis 30 und
x eine Zahl von 2 bis 20 ist,

B -S- , 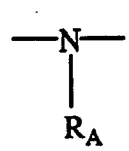 oder 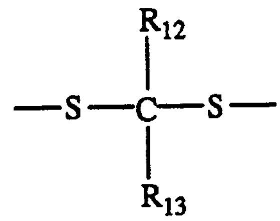 ist, worin

$R_A$ $C_1$-$C_{20}$-Alkyl, Phenyl oder mit einer oder mehreren Alkylgruppen mit insgesamt 1 bis 20 C-Atomen substituiertes Phenyl, Cyclohexyl oder

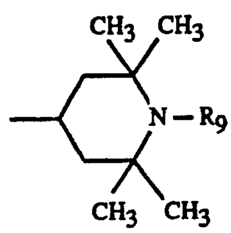

ist, worin $R_9$ die genannte Bedeutung hat, und
$R_{12}$ und $R_{13}$, unabhängig voneinander, Wasserstoff, $C_1$-$C_{12}$-Alkyl oder Phenyl sind oder
$R_{12}$ und $R_{13}$, mit dem C-Atom, an das sie gebunden sind, einen Cycloalkylring mit 5 bis 12 C-Atomen bilden, und
$b_1$ eine Zahl von 2 bis 10 ist,
oder,
wenn n = 3 ist,

A 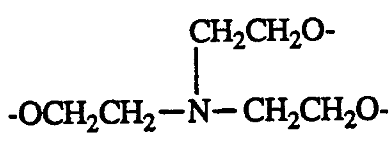 , 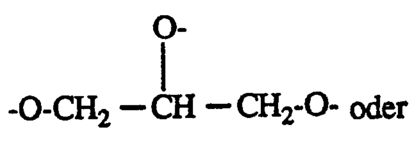 oder 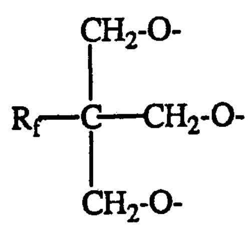

ist,
worin
$R_f$ $C_1$-$C_{24}$-Alkyl oder Phenyl ist,
oder,
wenn n = 4 ist,

A 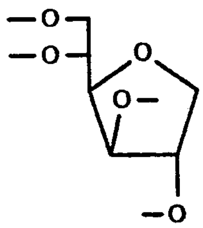 , 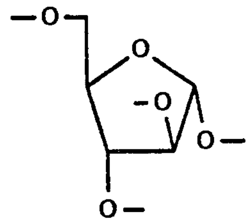 oder 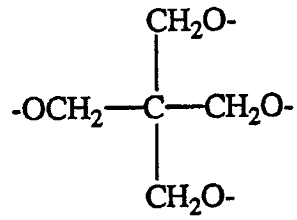

ist,
oder,
wenn n = 6 ist,

A 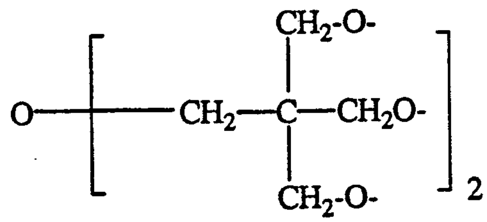

ist,
durch Umsetzung von Verbindungen der Formel II

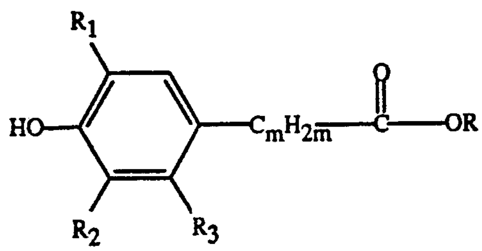 (II),

wobei m, $R_1$, $R_2$ und $R_3$ die obengenannte Bedeutung haben, und R Alkyl mit 1 bis 4 Kohlenstoffatomen ist, mit Verbindungen der Formel III

$$A(-H)_n \qquad (III),$$

wobei A und n die obengenannte Bedeutung haben, dadurch gekennzeichnet, dass man die Reaktion in Gegenwart eines Katalysators ausführt, der als aktives Material eine Alkalimetallverbindung der Formel IV

$M_mAn$ (IV)

worin
M Li, Na, K, Rb oder Cs,
m die Wertigkeit von An und
An einen Fluorid-, Hydroxid-, Phosphat-, Formiat-, Acetat- oder $-OR_5$-Rest ist, und $R_5$ $C_1$-$C_4$-Alkyl oder Phenyl ist,
und als Träger ein alkalisches Material, das gemessen in 10 Gew.-%-iger wässriger Suspension, einen pH-Wert von grösser als 10 aufweist, ausgewählt aus einer oder mehreren Substanzgruppen der Reihe der Erdalkalimetalloxide, -hydroxide, -aluminate oder -silikate, enthält.

**2.** Verfahren nach Anspruch 1, dadurch gekennzeichnet, dass Verbindungen der allgemeinen Formel Ia

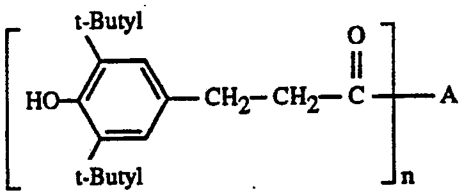

(Ia),

wobei A und n die in Anspruch 1 angegebene Bedeutung haben, durch Umsetzung einer Verbindung der Formel IIa

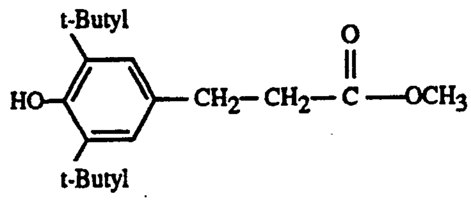

(IIa),

mit einer Verbindung der Formel III nach Anspruch 1 in Gegenwart eines Katalysators nach Anspruch 1 hergestellt werden.

3. Verfahren nach Anspruch 1, dadurch gekennzeichnet, dass der Katalysator die Oxide, Hydroxide, Aluminate oder Silikate der Erdalkalimetalle Mg, Ca, Sr und Ba oder deren Gemische als Träger enthält.

4. Verfahren nach Anspruch 1, dadurch gekennzeichnet, dass der Katalysator als aktives Material Hydroxide oder Fluoride der Alkalimetalle Na, K, Rb oder Cs enthält.

5. Verfahren nach Anspruch 1, dadurch gekennzeichnet, dass der Katalysator 0,15 bis 30 Gew.-%, berechnet auf das reine Alkalimetall und bezogen auf den wasserfreien Träger, aktives Material enthält.

6. Verwendung der Katalysatoren nach Anspruch 1 in einem Verfahren zur Umsetzung einer Verbindung der Formel II mit einer Verbindung der Formel III zu einer Verbindung der Formel I, wobei in den Formeln I,II und III angegebenen Substituenten die in Anspruch 1 angegebene Bedeutung haben.

7. Verfahren zur Herstellung von Verbindungen der allgemeinen Formel V

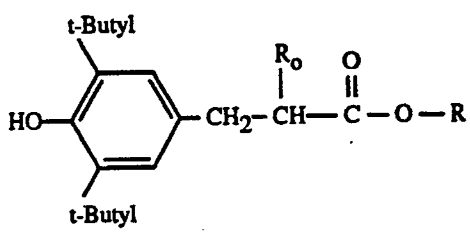

(V),

worin
$R_0$ Wasserstoff oder $C_1$-$C_4$-Alkyl, insbesondere Wasserstoff oder Methyl, und
R $C_1$-$C_4$-Alkyl ist,
durch Umsetzung von 2,6-Di-tert.-butylphenol mit einem Acrylsäureester der Formel VI

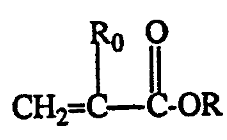

(VI),

worin $R_0$ und R die obengenannten Bedeutungen haben, dadurch gekennzeichnet, dass die Umsetzung in Gegenwart eines Katalysators ausgeführt wird, der als aktives Material eine Komponente der Formel IV

$M_mAn$ (IV)

worin
M Na, K, Rb oder Cs,
m die Wertigkeit von An und
An ein Fluorid-, Hydroxid- oder $-OR_5$-Rest ist, und $R_5$ $C_1$-$C_4$-Alkyl oder Phenyl ist, und als Träger ein alkalisches Material, das, gemessen in 10-Gew.-%-iger wässriger Suspension, einen pH-Wert von grösser als 10 aufweist, ausgewählt aus einer oder mehreren Substanzgruppen aus der Reihe der hydratisierten Erdalkalimetalloxide, -aluminate oder -silikate, enthält.

8. Verfahren nach Anspruch 7, dadurch gekennzeichnet, dass der Katalysator 0,15 bis 30 Gew.-%, berechnet auf das reine Alkalimetall und bezogen auf den Träger, aktives Material enthält.

9. Verfahren nach Anspruch 7, dadurch gekennzeichnet, dass der Katalysator einen Träger enthält, dessen Carbonatgehalt unter 0,1 Gew.-% und dessen Gehalt an Eisen unter 10 ppm liegt.

10. Verfahren nach Anspruch 7, dadurch gekennzeichnet, dass der Katalysator in Mengen von 0,1 bis 10 Mol-% aktivem Material, bezogen auf 2,6-Di-tert.-butylphenol, angewendet wird.

11. Verfahren zur Herstellung von Verbindungen der allgemeinen Formel VII

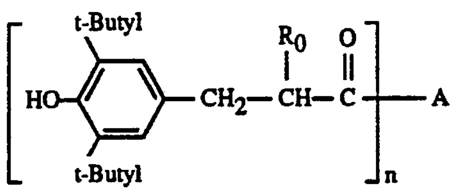

(VII),

worin
$R_0$ Wasserstoff oder $C_1$-$C_4$-Alkyl, insbesondere Wasserstoff oder Methyl, und
n 1, 2, 3, 4 oder 6 ist, worin,
wenn n = 1 ist,
A $-OR_4$ ist, worin
$R_4$ $C_2$-$C_{45}$-Alkyl, $C_5$-$C_{12}$-Cycloalkyl, $C_2$-$C_{18}$-Alkenyl,

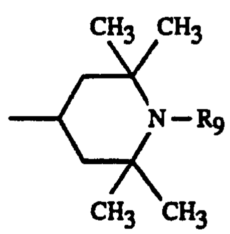

oder $-CH_2CH_2-XR_{5a}$ ist, worin
$R_9$ Wasserstoff, $C_1$-$C_8$-Alkyl,

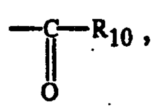

$-O^{\cdot}$ oder $-OR_{9'}$ ist, worin

$R_{9'}$ Wasserstoff oder $C_1$-$C_{25}$-Alkyl oder

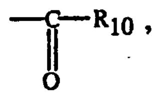

und
$R_{10}$ Wasserstoff oder Alkyl mit 1 bis 20 C-Atomen ist,
X -O-, -S- oder

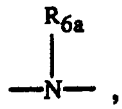

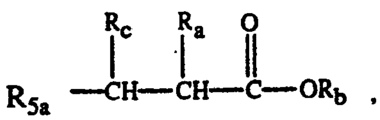

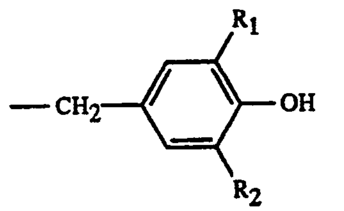

Wasserstoff
$C_1$-$C_{24}$-Alkyl, Phenyl, $C_5$-$C_{12}$-Cycloalkyl oder

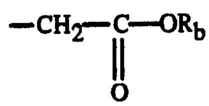

ist, worin
$R_a$ Wasserstoff oder Methyl,
$R_b$ Wasserstoff oder $C_1$-$C_{24}$-Alkyl und
$R_c$ Wasserstoff oder Methyl ist, mit der Massgabe, dass $R_a$ und $R_c$ nicht gleichzeitig Methyl sind,
und
$R_{6a}$ $C_1$-$C_{18}$-Alkyl, Phenyl, mit einer oder mehreren Alkylgruppen mit insgesamt 1 bis 24 C-Atomen substituiertes Phenyl oder $C_5$-$C_8$-Cycloalkyl ist,
oder,
wenn n = 2 ist,
A $-O-C_xH_{2x}-O-$,
$-O-(CH_2CH_2O)_aCH_2CH_2O-$,
$-O-CH_2-CH_2-B-CH_2CH_2O-$,

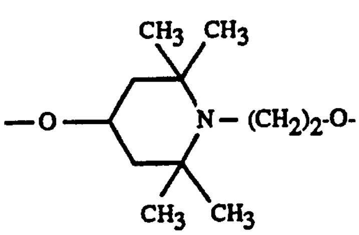 ,

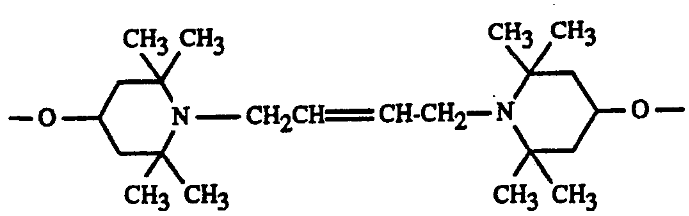

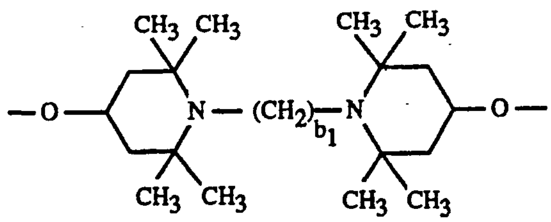

$-O-CH_2CH=CHCH_2-O-$, $-O-CH_2C\equiv CCH_2-O-$,

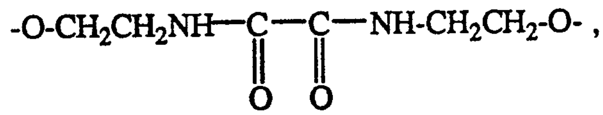

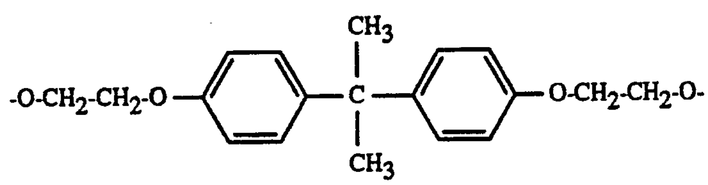 oder 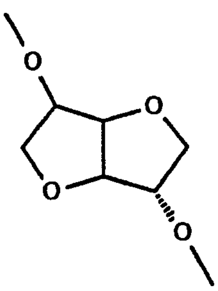

ist,
worin
a eine Zahl von 1 bis 30,
x eine Zahl von 2 bis 20 ist,
B -S- ,

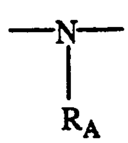 oder 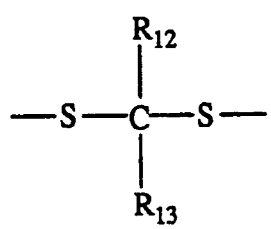

ist, worin
$R_A$ $C_1$-$C_{20}$-Alkyl mit 1 bis 20 C-Atomen, Phenyl oder mit einer oder mehreren Alkylgruppen mit insgesamt 1 bis 20 C-Atomen substituiertes Phenyl, Cyclohexyl oder

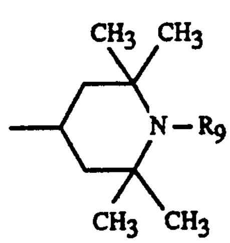

ist, wobei $R_9$ vorstehende Bedeutung hat, und

$R_{12}$ und $R_{13}$, unabhängig voneinander, Wasserstoff, $C_1$-$C_{12}$-Alkyl oder Phenyl sind oder
$R_{12}$ und $R_{13}$, mit dem C-Atom, an das sie gebunden sind, einen Cycloalkylring mit 5 bis 12 C-Atomen bilden, und
$b_1$ eine Zahl von 2 bis 10 ist,
oder,
wenn n = 3 ist,

A 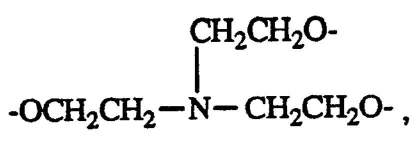 , 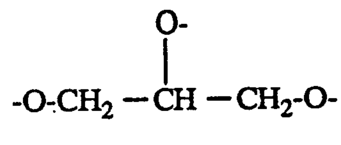 oder 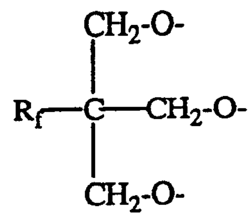

ist,
worin
$R_f$ $C_1$-$C_{24}$-Alkyl oder Phenyl darstellt,
oder,
wenn n = 4 ist,

A 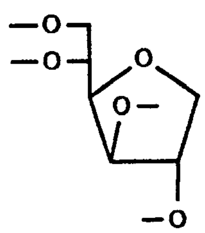 , 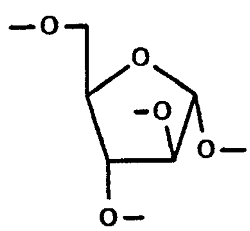 oder 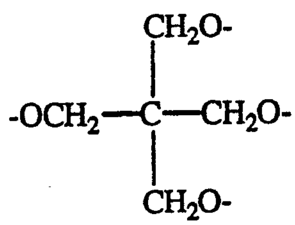

ist,
oder,
wenn n = 6 ist,

A 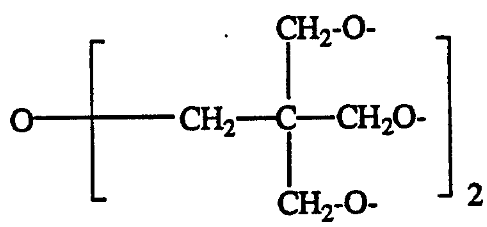

ist,
dadurch gekennzeichnet, dass man eine Verbindung der Formel VI

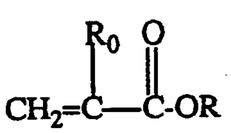

(VI),

worin $R_0$ Wasserstoff oder $C_1$-$C_4$-Alkyl und R $C_1$-$C_4$-Alkyl ist, mit 2,6-Di-tert.-butylphenol in erster Stufe zu einer Verbindung der Formel V umsetzt und letztere, ohne sie aus dem Reaktionsgemisch zu isolieren, in einer zweiten Stufe mit einer Verbindung der Formel III

$A(-H)_n$ (III),

wobei A und n die genannte Bedeutung haben, umsetzt, wobei beide Stufen in Gegenwart eines Katalysators durchgeführt werden, der als aktives Material eine Komponente der Formel IV

$M_mAn$ (IV)

worin
M Na, K, Rb oder Cs,
m die Wertigkeit von An und
An ein Fluorid-, Hydroxid- oder $-OR_5$-Rest ist, und $R_5$ $C_1$-$C_4$-Alkyl oder ein Phenyl ist,

und als Träger ein alkalisches Material, das, gemessen in 10 Gew.-%-iger Suspension, einen pH-Wert von grösser als 10 aufweist, ausgewählt aus einer oder mehreren Substanzgruppen aus der Reihe der hydratisierten Erdalkalimetalloxide, -aluminate oder -silikate, enthält.

**12.** Verwendung der Katalysatoren nach Anspruch 7 in einem Verfahren zur Umsetzung einer Verbindung der Formel VI mit 2,6-Di-tert.-butylphenol zu einer Verbindung der Formel V.

**13.** Verwendung der Katalysatoren nach Anspruch 7 bei der Umsetzung einer Verbindung der Formel VI mit 2,6-Di-tert-butylphenol und der anschliessenden Umsetzung einer Verbindung der Formel V ohne Zwischenisolierung mit einer Verbindung der Formel III

$$A \leftarrow H)_n .$$